# EUROPÄISCHE PATENTSCHRIFT

(11) **EP 3 071 242 B1**
(45) Veröffentlichungstag und Bekanntmachung des Hinweises auf die Patenterteilung: **11.09.2019**
(21) Anmeldenummer: 14835694.2
(22) Anmeldetag: 11.11.2014
(51) Int. Cl.: A61L 2/18, A61L 2/20, C02F 1/72, C02F 1/78, F28F 25/00, C02F 103/02

(54) **VERFAHREN ZUR OXIDATIVEN BEHANDLUNG VON EINER FLÜSSIGPHASE UND/ODER EINER GASPHASE UND/ODER EINER FESTPHASE**
METHOD FOR THE OXIDATIVE TREATMENT OF A LIQUID PHASE AND/OR A GAS PHASE AND/OR A SOLID PHASE
PROCÉDÉ DE TRAITEMENT OXYDATIF D'UNE PHASE LIQUIDE ET/OU D'UNE PHASE GAZEUSE ET/OU D'UNE PHASE SOLIDE

(30) Priorität: 20.11.2013 DE 102013019339
(43) Veröffentlichungstag der Anmeldung: 28.09.2016
(73) Patentinhaber: Nonnenmacher, Klaus, 72070 Tübingen (DE)
(72) Erfinder: Nonnenmacher, Klaus, 72070 Tübingen (DE)
(74) Vertreter: Eberle, Michael
(86) Internationale Anmeldenummer: PCT/DE2014/000573
(87) Internationale Veröffentlichungsnummer: WO 2015/074638

(56) Entgegenhaltungen:
- GB-A- 1 278 043
- GB-A- 2 468 518
- US-A- 4 172 786
- US-A1- 2003 047 521
- US-A1- 2006 233 683
- FEHN J ET AL: "OXIDATION BIOLOGISCH SCHWER ABBAUBARER STOFFE MIT OZON", GWF WASSER ABWASSER, 971956 2, Bd. 136, Nr. 6, 1. Juni 1995 (1995-06-01), Seiten 302-310, XP000514300, ISSN: 0016-3651

## Beschreibung

Die vorliegende Erfindung betrifft ein Verfahren zur oxidativen Behandlung von einer Flüssigphase und/oder einer Gasphase und/oder einer Festphase. Mit dem Verfahren kann beispielsweise belastetes Wasser aufbereitet werden.

Eine oxidative Behandlung von organisch belastetem Wasser ist ein wesentlicher Bestandteil der Wasseraufbereitung, beispielsweise in Zusammenhang mit einer Abwasserreinigung. Neben biologischen und physikalischen Reinigungsverfahren dient die oxidative Behandlung von Wasser zur Beseitigung belastender Substanzen im Wasser. Der Gebrauch von Wasser führt zu den verschiedensten Verunreinigungen. Beispiele hierfür sind Pestizide, Herbizide, Brandverhütungsmittel, Weichmacher oder Pharmazeutika. Weitere Quellen für Verunreinigungen sind Schwimmbäder, Papierfabriken oder auch die Trinkwasserchlorierung. So gelangen beispielsweise verschiedene halogenierte organische Kohlenwasserstoffe als Spurenschadstoffe in den Wasserkreislauf. Wichtige Vertreter dieser Schadstoffe werden als AOX-Substanzen (adsorbierbare organisch gebundene Halogene) zusammengefasst, wobei hiervon Chlor-, Brom- und lodverbindungen umfasst werden. Derartige Substanzen sind oftmals gesundheitsgefährdend. Andere belastende Substanzen in Wasser sind Bakterien, Viren, Pilze, Pilzsporen oder deren Bestandteile, von denen ebenfalls eine gesundheitliche Gefährdung ausgehen kann. Zur Entfernung von diesen Verunreinigungen ist eine aufwendige Wasserreinigung und -aufbereitung erforderlich. Die Integration von oxidativen Behandlungsprozessen in die Kläranlagenabläufe ist dabei ein wichtiger Bestandteil.

Zum Abbau von Schadstoffen und zur Desinfektion oder Entkeimung von Wasser ist eine oxidative Behandlung mit Ozongas bekannt. Ozon wirkt hierbei als Oxidationsmittel. In Kombination beispielsweise mit UV-Strahlung oder mit Wasserstoffperoxid (H₂O₂) kann die Effizienz des oxidativen Prozesses weiter gesteigert werden (Hoigné, Jürg (1998),,Chemistry of aqueous Ozone and Transmission of Pollutants by Ozonation and advanced oxidation processes, in: Hrubec, Jifi (1998): Quality and Treatment of Drinking Water II. Berlin, Heidelberg: Springer (The Handbook of Environmental Chemistry, Part C, 5/5 C), Seiten 83 -141.)

Ozon wird im Allgemeinen gasförmig technisch hergestellt. Es löst sich physikalisch in Wasser und reagiert nicht mit dem Wasser. Für die oxidative Wirkung des Ozons ist insbesondere das Hydroxylradikal •OH verantwortlich. Eine Vorstufe (Prekursor) des Hydroxylradikals ist das Hydroperoxid-Anion HO₂⁻, das bei einem Ozonzerfall gemäß derfolgenden Reaktionsgleichung entsteht:

(G1) O₃ + OH⁻ → HO₂⁻ + O₂

Die Umsetzungsrate ist auch bei höheren pH-Werten nur moderat. Die Halbwertszeit beträgt bei pH 8 beispielsweise circa 2,8 Stunden.

Bei der Dissoziation von Wasserstoffperoxid entsteht ebenfalls das Hydroperoxid-Anion:

(G2) H₂O₂ ←→ HO₂⁻ + H⁺

Der pKa-Wert von Wasserstoffperoxid liegt bei 11,6, so dass bei höheren pH-Werten das Gleichgewicht auf die Seite von HO₂⁻ verschoben wird. Dennoch bleibt die Prekursor-Bildung moderat.

Durch eine Zugabe von Wasserstoffperoxid in Ozonwasser, also in Wasser mit gelöstem Ozon, kann eine weitere Quelle für den Prekursor bereitgestellt werden. Zusammengefasst ergibt sich die folgende Reaktion mit Ozon und Wasserstoffperoxid:

(G3) 2O₃ + H₂O₂ → 2 •OH + 3O₂

Auch diese Gesamtreaktion verläuft nur moderat, so dass eine oxidative Wasserbehandlung mit Ozon und Wasserstoffperoxid oftmals keine befriedigenden Resultate erzielt.

Eine Bildung von Hydroxylradikalen ist auch durch das Ozonid-Anion O₃⁻ möglich, das bei einer Ozonisierung von Aromaten gemäß den folgenden Gleichungen entsteht:

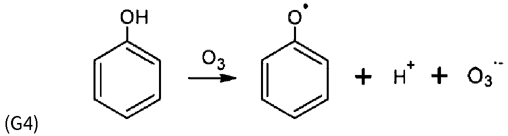

(G5) O₃⁻ + H₂O → •OH + O₂ + OH⁻

Untersuchungen zeigen, dass bei einer organischen DOM-Matrix (DOM - dissolved organic matter) in bestimmten Fällen eine unerwartet hohe Abbaurate von PCBA(4- Chlorobenzoesäure), das ein Zwischenprodukt beim bakteriologischen Abbau von Herbiziden ist, mit einer kombinierten Ozon- und Wasserstoffperoxidbehandlung zu erreichen ist (von Sonntag, Clemens; von Gunten, Urs (2012) Chemistry of Ozone in Waterand Waste Water Treatment. London: IWA Publishing). Hierbei kann die organische Matrix aliphatisch sein oder eine Mischung von aromatischen und aliphatischen Substanzen zeigen. Der Anteil der Matrix, der bei der reinen Ozonisierung eine Hydroxylradikal-Bildung begünstigt, wird hierbei lediglich hydroxyliert (R-COOH), jedoch nicht vollständig zu CO₂ abgebaut. Daher reduziert sich der Gehalt an gelöster organischer Struktur (DOC = dissolved organic carbon) nicht wesentlich. Die peroxidische DOM-Matrix (R-COOH) erzeugt jedoch weiterhin Hydroxylradikale. Diese bleiben bestehen und beeinflussen die Stabilität des gelösten Ozons im Wasser. Eine solche Hydroxylradikal-Bildung ist zwar erwünscht. Die Wasserbehandlung sollte jedoch eine vollständige Entfernung der gelösten organischen Kohlenstoffe erbringen.

Aus der US 2006/0233683 A1 ist es bekannt, zwecks Bildung von Hydroxylradikalen Olefine mit ozonhaltigem Sauerstoff zu versetzen. Hierbei können in situ auch Ozonide entstehen.

Aus der GB 1278043 A ist es bekannt, einen Olefindampf mit Ozon zu kontaktieren. Auch hierbei können in situ Ozonide entstehen. Die Kontaktierung der Komponenten erfolgt wasserfrei.

Aus der GB 2468518 A ist ein Sterilisationsverfahren unter Verwendung von Ozon bekannt. Beschrieben wird u.a. das Einbringen von Ozon in einen Kanal, in dem auch ein Cycloalken eingespeist werden kann. Auch hierbei können in situ Ozonide entstehen.

Aus der US 4172786 A ist ein Verdunstungskühler bekannt, durch den ozonhaltiges Wasser zirkuliert.

Der vorliegenden Erfindung liegt demgegenüber die Aufgabe zugrunde, ein neues Verfahren zur oxidativen Behandlung von belastetem Wasser bereitzustellen, bei dem die Reinigungs- und Desinfektionswirkung der oxidativen Behandlung verbessert und effektiver wird. Zugleich soll das Verfahren mit verhältnismäßig wenig Aufwand und Kosten in der Praxis realisierbar sein. Ein weiterer Aspekt der Erfindung ist die Behandlung von Abgasen oder Abluft oder Feststoffen, die beispielsweise mit geruchsintensiven Verbindungen oder anderen Verunreinigungen oder Kontaminationen belastet sind.

Diese Aufgabe wird durch ein Verfahren zur oxidativen Behandlung einer Flüssigphase und/oder einer Gasphase und/oder einer Festphase gelöst, wie es sich aus dem Anspruch 1 ergibt. Bevorzugte Ausgestaltungen dieses Verfahrens sind Gegenstand der weiteren Ansprüche.

Das erfindungsgemäße Verfahren basiert darauf, dass die zu behandelnden Flüssigkeiten, beispielsweise belastetes Wasser, oder die zu behandelnden Gase, beispielsweise Abgase, oder die zu behandelnden Feststoffe einer oxidativen Kombinationsbehandlung unterzogen werden, wobei für die Behandlung einerseits Ozon und andererseits eine Komponente, die durch die Ozonisierung von einem Olefin bereitgestellt wird, verwendet werden. Bei dieser Komponente, die im Folgenden auch verallgemeinernd als Ozonidkomponente bezeichnet wird, handelt es sich vorzugsweise um ein organisches Ozonid und/oder um ein organisches Peroxid und/oder um Mischungen davon, die für die Behandlung bereitgestellt werden. In allen drei Fällen wird hierdurch eine reinigende und/oder desinfizierende Wirkung erzielt. Die Zugabe des Ozons und die Zugabe des ozonisierten Olefins oder gegebenenfalls von Vorläufermoleküle hiervon kann gleichzeitig oder im Prinzip in beliebiger Reihenfolge erfolgen. Prinzipiell kann die Zugabe der erforderlichen Substanzen in flüssiger und/oder gasförmiger und/oder dampfförmiger und/oder sprühnebeliger Form erfolgen. Dieses Verfahren zur oxidativen Behandlung ist für die Behandlung von Flüssigphasen, von Gasphasen und von Festphasen (Feststoffen) geeignet, wobei das Verfahren erfindungsgemäß in Gegenwart von Wasser durchgeführt wird. Das Wasser kann dabei beispielsweise in flüssiger Form, gasförmig und/oder in Form eines Aerosols zugesetzt werden. Für eine Behandlung von Gasen und für eine Behandlung von Feststoffen ist beispielsweise eine Befeuchtung (Benebelung) vorgesehen. Die im Zuge des erfindungsgemäßen Verfahrens ausgelösten radikalischen Reaktionen laufen dabei immer im Prinzip in einer wässrigen Phase ab, wodurch die chemischen Reaktionen in allen drei Fällen (Flüssigphase, Gasphase, Festphase) vergleichbar sind. Die Ozonidkomponente, das Ozon und das Wasser reagieren dabei so, dass Hydroxylradikale entstehen, die der Ausgangspunkt für eine Kettenreaktion sind. Bei der Kettenreaktion reagieren unselektiv organische Bestandteile der zu behandelnden Phase bis zu deren höchster Oxidationsstufe (CO₂). Dabei werden wieder Hydroxylradikale gebildet, solange organische Substanz vorhanden ist.

Der Kern der Erfindung ist die kombinierte Behandlung einer Flüssigphase, Gasphase und/oder Festphase mit Ozon und mit wenigstens einer Komponente, die durch die Ozonisierung von wenigstens einem Olefin bereitgestellt wird. Mit dem Ausdruck Olefin (oder auch Alken) sind in diesem Zusammenhang allgemein ungesättigte Kohlenwasserstoffverbindungen zu verstehen. Diese Verbindungen können sowohl linear als auch cyclisch sein. Wesentlich ist hierbei, dass die Verbindungen wenigstens eine Kohlenstoff-Kohlenstoff-Doppelbindung aufweisen. Es handelt sich also um ungesättigte Kohlenwasserstoffverbindungen oder, mit anderen Worten, um eine organische Substanz mit einem ungesättigten Kohlenwasserstoffgerüst. Vorzugsweise handelt es sich bei dem wenigstens einen Olefin um eine oder mehrere ungesättigte Fettsäuren. Für die Zwecke der Erfindung können jedoch auch andere Verbindungen eingesetzt werden, beispielsweise reine Kohlenwasserstoffe, die wenigstens eine Kohlenstoff-Kohlenstoff-Doppelbindung aufweisen.

Die Komponente, die durch die Ozonisierung von wenigstens einem Olefin bereitgestellt wird, wird in einem separaten Prozess hergestellt, wobei ein Olefin mit Ozon behandelt wird, und für die oxidative Behandlung bereitgestellt wird. Die Ozonisierung der Olefine kann in Gegenwart von Wasser (protisch) oder in Abwesenheit von Wasser (aprotisch) erfolgen, wobei unter protischen Bedingungen schwerpunktmäßig organische Peroxide und unter aprotischen Bedingungen schwerpunktmäßig stabile organische Ozonide gebildet werden. Die Reaktionsbedingungen für die Ozonisierung der Olefine werden vorzugsweise in Abhängigkeit von dem Taupunkt des eingesetzten Trägergases gewählt. Wenn beispielsweise trockenes Ozongas als Trägergas eingesetzt wird, kann die Ozonisierung der Olefine vorzugsweise aprotisch durchgeführt werden. Je nach Reaktionsbedingungen bei der Ozonisierung können unterschiedliche Produkte entstehen. Beispielsweise bei aprotischen Bedingungen können monomere und/oder polymere Ozonide, beispielsweise 1,2,4-Trioxolan, gebildet werden. Insbesondere bei der Verwendung von protischen Lösungsmitteln bei der Ozonisierungsreaktion können auch peroxidische Verbindungen, insbesondere organische Hydroperoxide oder Mischungen der genannten Substanzen, gebildet werden, beispielsweise polymere oder dimere Peroxide wie z.B. 1,2,4,5-Tetroxan.

Gemäß der Erfindung erfolgt die Herstellung des organischen Ozonids aprotisch in einem separaten Prozess.

Durch die Ozonisierung der Olefine werden also, je nach Reaktionsbedingungen, organische Ozonide und/oder organische Peroxide und/oder Hydroperoxide oder Mischungen davon bereitgestellt, die für das erfindungsgemäße Verfahren genutzt werden. Die resultierende Substanz, die im Folgenden allgemein als Ozonidsubstanz bezeichnet wird, wird gemäß der Erfindung als ölige oder cremeartige Substanz in das Wasser eingebracht. Beim Kontakt mit dem Wasser zerfällt das organische Ozonid in die Bruchstücke Aldehyd und Carbonsäure. Hierbei bildet sich Wasserstoffperoxid bzw. das Hydroperoxid-Anion, das ein Prekursor des Hydroxylradikals ist. In der Gegenwart von Ozon bildet sich durch den Kontakt des Ozonids mit Wasser damit letztendlich das Hydroxylradikal, das dann für die oxidativen Prozesse zum Abbau der Schadstoffe zur Verfügung steht. Das Wasserstoffperoxid bewirkt weiterhin eine Mineralisierung der Bruchstücke des Ozonids, also der Carbonsäure und des Aldehyds, in Kohlendioxid und Wasser.

Die Komponente, die durch die Ozonisierung der Olefine hergestellt wird, wird vorteilhafterweise in adsorbierter Form für den Prozess bereitgestellt, beispielsweise durch Adsorption an einer Kieselsteinpackung oder an anderen Oberflächen, beispielsweise an metalloxidischen oder keramischen Materialen oder an Glas. Allgemein eignen sich hierfür insbesondere Kieselsteine, Glaskörper, Glashohlkörper, Keramik, beispielsweise Kaolin, Kunststoffe, Textilien oder Metalle, beispielsweise in Form von Vliesen, Waben, Rohren, Kugeln, Hohlkörpern oder Schüttungen. Zweckmäßigerweise stellt die Packung eine fettige, also hydrophobe Oberfläche für die Adsorption der Ozonidkomponente bereit. Insbesondere für die oxidative Behandlung von Wasser oder von Gasen wird eine Packung für eine Adsorption der Komponente mit den ozonisierten Olefinen bereitgestellt, die vorzugsweise stromabwärts einer Dosierungsstelle für die Ozonidkomponente liegt. Eine entsprechende Packung kann beispielsweise in einem Rohrreaktor und/oder am Grund eines Beckens vorgesehen sein. Die Effektivität der Behandlung kann durch oxidische Oberflächen bei diesen Materialien gesteigert werden.

Das erfindungsgemäße Verfahren ist insbesondere zur oxidativen Behandlung von belastetem Wasser als Flüssigphase, insbesondere von organisch belastetem Wasser, geeignet. Schwerpunkt der Anwendung des erfindungsgemäßen Verfahrens ist die Behandlung von Abwässern. Im Prinzip kann dieses Verfahren auch zur oxidativen Behandlung von anderen Flüssigkeiten, insbesondere von wässrigen Flüssigkeiten, eingesetzt werden. Zur oxidativen Behandlung wird das Wasser ozonisiert, also mit Ozon versetzt. Im Allgemeinen löst sich das Ozon hierbei im Wesentlichen physikalisch im Wasser.

Mit diesem Verfahren kann organisch belastetes Wasser zuverlässig aufbereitet werden, so dass das Wasser beispielsweise wieder einem natürlichen Gewässer zugeführt werden kann oder für die Bereitstellung von Trinkwasser verwendet werden kann. Mit dem erfindungsgemäßen Verfahren können vor allem Spurenschadstoffe, die im Wasserkreislauf akkumulieren können, zuverlässig beseitigt werden. Darüber hinaus wird durch die erfindungsgemäße oxidative Behandlung eine Desinfektion des Wassers insbesondere zur Beseitigung von bakteriologischen Belastungen erreicht. Untersuchungen des Erfinders haben gezeigt, dass mit dieser kombinierten oxidativen Behandlung ein unerwartet hoher reinigender Effekt erzielt wird. Dies äußert sich beispielsweise darin, dass der Ozonverbrauch für den Abbau einer bestimmten Menge an organische Substanz etwa um den Faktor 10 sinkt. Der besondere reinigende Effekt der erfindungsgemäßen Kombinationsbehandlung lässt sich folgendermaßen erklären. Durch die Behandlung des ozonisierten Wassers, beispielsweise mit einer ozonisierten Fettsäure als Olefin, wird das im Wasser physikalisch gelöste Ozon (03) letztendlich zu Hydroxylradikalen umgewandelt. Die Hydroxylradikale bewirken die Oxidation der verschiedenen, im Wasser enthaltenen Substanzen und führen letztendlich zu einer Mineralisierung und damit zu einem Abbau der belastenden organischen Substanzen. Das ozonisierte Olefin und die bei diesem Prozess gebildeten Produkte sind hierbei Initiatoren einer radikalischen Kettenreaktion, die zu einer Entfernung der Schadstoffe und Kontaminationen aus dem Wasser führt.

Das Ozon für das erfindungsgemäße Verfahren wird in bevorzugter Weise vor Ort hergestellt, beispielsweise mittels eines Ozongenerators, bei dem das Ozon durch eine Plasmaentladung (Barriereentladung) elektrisch hergestellt wird. Als Trägergas für die Ozonherstellung kann beispielsweise trockener Sauerstoff mit einem Sauerstoffgehalt von etwa 99% eingesetzt werden. Der Taupunkt des trockenen Sauerstoffs liegt dabei insbesondere unterhalb von -60°C. Weiterhin ist es beispielsweise möglich, für die Bereitstellung von Sauerstoff ein Molekularsieb einzusetzen, um aus der Umgebungsluft angereicherten Sauerstoff (circa 90%) zu gewinnen. Der Taupunkt des Sauerstoffs liegt hierbei bei -20°C oder niedriger. Es kann im Prinzip auch feuchter Sauerstoff oder atmosphärische Luft als Trägergas eingesetzt werden, wobei im Allgemeinen der Wirkungsgrad bei der Ozonbildung mit trockenem Sauerstoff größer ist.

Die Ozonisierung der Olefine zur Bereitstellung der Ozonidkomponente für die oxidative Behandlung des Wassers kann ebenfalls vor Ort durchgeführt werden. Es ist auch möglich, dass die Ozonisierung der Olefine an anderer Stelle durchgeführt wird und dass die hierbei resultierende Substanz, also die oben bereits erwähnte Ozonidsubstanz, zum Verbrauchsort transportiert wird. Dies ist möglich, da die Ozonidsubstanz im Allgemeinen stabil und lagerfähig ist. Die Herstellung der Komponente kann chargenweise oder kontinuierlich erfolgen.

Erfindungsgemäß wird die Ozonisierung der Olefine aprotisch durchgeführt. Für den Herstellungsprozess wird beispielsweise Ozongas, das insbesondere aus dem oben beschriebenen Ozongenerator stammt, als Trägergas eingesetzt. Vorzugsweise wird trockenes Ozongas verwendet. Die Verwendung von trockenem Ozongas bei der Ozonisierung der Olefine ist einfach handhabbar und liefert gute Ozonisierungsausbeuten. Die wesentlichen Reaktionsprodukte sind hierbei das bereits erwähnt Trioxolan (Ozonid) und/oder das Tetroxan. Die resultierende Substanz ist über einen längeren Zeitraum lagerfähig. Die Substanz ist im Wesentlichen nicht wasserlöslich, insbesondere wenn ein hoher Anteil an cis-Ozoniden gebildet wird. Zur Vereinfachung wird die Substanz, die bei der Ozonisierung der Olefine gebildet wird, im Folgenden zum Teil einfach als Ozonidsubstanz bezeichnet, wobei diese Substanz nicht zwingend ein oder mehrere Ozonide enthalten muss. Es kann auch der Fall sein, dass organische Peroxide oder Hydroperoxide oder Mischungen von peroxidischen und/oder hydroperoxidischen Substanzen und von Ozoniden enthalten sind. Die gebildete Ozonidsubstanz liegt als ölige oder cremige Substanz vor, die direkt für das erfindungsgemäße Verfahren eingesetzt werden kann.

Zweckmäßigerweise werden ein- oder mehrfach ungesättigte Fettsäuren als Ausgangssubstanzen für die Ozonisierung der Olefine verwendet. Besonders geeignet ist beispielsweise Ölsäure oder Leinöl oder Gemische verschiedener Fettsäuren. Die Fettsäuren können in bevorzugter Weise in Form von Pflanzenölen bereitgestellt werden. Die Ölsäure kann beispielsweise in reiner Form oder, in einer besonders bevorzugten Ausgestaltung, als Olivenöl, insbesondere als natives Olivenöl, das circa 70% Ölsäure enthält, bereitgestellt werden.

Der Reaktionsansatz bei der Ozonisierung der Olefine kann mit einem geeigneten Lösungsmittel verdünnt werden, beispielsweise mit Dimethylsulfoxid (DMSO). Im Prinzip können bei der Ozonisierung der Olefine als Lösungsmittel Wasser und/oder beispielsweise RNH2, ROOC und/oder ROH eingesetzt werden.

Die aprotische Ozonidbildung erfolgt nach dem sogenannten Criegee-Mechanismus gern der nachfolgenden Gleichung, wobei das Ozonid an der Doppelbindung der Olefine (in diesem Beispiel Ölsäure) entsteht:

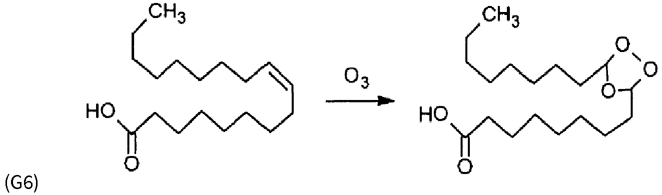

Das hierbei gebildete Ozonid ist als Sauerstoffringverbindung sehr beständig. Es ist daher möglich, das Ozonid zu lagern und je nach Bedarf einzusetzen. Das Criegee-Ozonid bildet eine cremeartige oder ölartige Substanz, die beispielsweise mit einer Zahnradpumpe tröpfchenweise in das Reaktionsbecken oder einen Rohrreaktor eingebracht werden kann.

Die Dosis der Komponente, die durch die Ozonisierung der Olefine bereitgestellt wird, insbesondere die Dosis der organischen Ozonidsubstanz, im Wasser liegt vorzugsweise zwischen ca. 0,001 ml / 100 m³ Wasser und ca. 100 ml / 100 m³ Wasser. In bevorzugter Weise wird beispielsweise soviel Ozonidsubstanz zugesetzt, dass die Konzentration im zu behandelnden Wasser etwa 1 ml Ozonidsubstanz pro 100 m³ Wasser beträgt. Diese Angaben gehen von der Annahme einer etwa gleichen spezifischen Dichte von Wasser und der flüssigen Ozonidsubstanz aus, wobei 1 ml Ozonidsubstanz (Lösung) etwa 1 g entspricht. Die genannten Mengen sind ausreichend, um eine Totaloxidation der im Wasser gelösten organischen Substanzen zu erreichen.

Die oxidative Behandlung des Wassers wird durch den pH-Wert des Wassers beeinflusst. Eine besonders effektive oxidative Behandlung kann in einem basischen Milieu erreicht werden. Vorteilhafter Weise wird der erfindungsgemäße Prozess bei einem pH-Wert oberhalb von pH 7, insbesondere bei pH 8 oder größer durchgeführt. Üblicherweise weisen Abwässer einen pH-Wert von größer als pH 7 auf, so dass für die Einstellung des pH-Wertes keine weiteren Maßnahmen ergriffen werden müssen. Wenn es erforderlich ist, kann der pH-Wert durch die Einleitung entsprechender Substanzen auf einen geeigneten pH-Wert eingestellt werden. Neben der Optimierung der oxidativen Prozesse hat ein pH-Wert im basischen Bereich weiterhin den Vorteil, dass hierdurch das Algenwachstum gehemmt wird. Insbesondere bei einem pH-Wert oberhalb von 8 verlässt das im Wasser enthaltene CO₂ das Wasser in gasförmiger Form, entsprechend dem Kalk-Kohlensäure-Gleichgewicht im Wasser, so dass das für eine Assimilation erforderliche CO₂ für die Algen nicht mehr zur Verfügung steht. Versuche haben jedoch gezeigt, dass selbst bei einem pH-Wert von circa 7 die oxidativen Prozesse bei dem erfindungsgemäßen Verfahren sehr effektiv ablaufen, so dass auf eine Einstellung des pH-Wertes auch durchaus verzichtet werden kann.

Das im Zuge des Prozesses gebildete Wasserstoffperoxid stellt eine weitere Quelle für die Hydroxylradikalbildung dar. Die oxidative Wirkung von Wasserstoffperoxid insbesondere durch die Bildung von Hydroxylradikalen kann durch eine UV-Bestrahlung während deroxidativen Behandlung verstärkt werden.

Anhand einer Messung von Wasserstoffperoxid im Wasser kann beurteilt werden, ob der oxidative Prozess abgeschlossen ist. Wasserstoffperoxid wird messbar, wenn keine Reaktionsmöglichkeit für das Wasserstoffperoxid mehr besteht. Die Wasserstoffperoxidkonzentration kann also als Indikator dafür verwendet werden, ob der Abbau der Spurenschadstoffe und Kontaminationen im Wasser vollständig erfolgt ist.

Wenn das gebildete Wasserstoffperoxid nach Abschluss des Prozesses nicht gewünscht ist, kann das überschüssige Wasserstoffperoxid anschließend entfernt werden. Hierfür kann insbesondere eine UV-Behandlung vorgesehen sein, beispielsweise eine UV-Bestrahlung mit einer Wellenlänge von 254 nm. Hierdurch wird das Wasserstoffperoxid letztendlich zu Wasser abgebaut. Die UV-Behandlung kann insbesondere am Ende des Wasserbehandlungsverfahrens durchgeführt werden, um vollständig unbelastetes Wasser bereitzustellen zu können.

Mit dem erfindungsgemäßen Verfahren kann ohne Weiteres eine vollständige Oxidation (Totaloxidation) der Spurenschadstoffe erreicht werden, so dass die in dem zu behandelnden Wasser enthaltenen gelösten organischen Substanzen vollständig eliminiert werden. Daher ist das erfindungsgemäße Aufbereitungsverfahren insbesondere für die Bereitstellung von Trinkwasser geeignet, da in Trinkwasser keine gelösten organischen Substanzen (kein gelöster organischer Kohlenstoff) mehr nachweisbar sein sollten. Im Vergleich mit herkömmlichen Verfahren kann bei dem erfindungsgemäßen Verfahren der Ozonaufwand im Verhältnis zum gelösten organischen Kohlenstoff (DOC - Dissolved Organic Carbon) erheblich reduziert werden. Bei herkömmlichen Verfahren beträgt das Verhältnis M(O₃) / M(DOC) circa 1 (M = Masse). Mit dem erfindungsgemäßen Verfahren kann ein Verhältnis M(O₃) / M(DOC) von circa 0,1 erreicht werden. Das bedeutet, dass der Ozonverbrauch um den Faktor 10 gesenkt werden kann. Die oxidative Ausnutzung des Ozons ist also bei dem erfindungsgemäßen Verfahren erheblich verbessert.

Um den Prozess optimal steuern zu können, werden die Prozessführungsparameter, insbesondere die Ozonkonzentration und die Wasserstoffperoxidkonzentration im Wasser, in einer bevorzugten Ausgestaltung des Verfahrens im Verlauf des Verfahrens gemessen. Vorzugsweise werden diese Parameter kontinuierlich gemessen. Auf der Basis dieser Messwerte kann die Ozondosierung und/oder die Ozonherstellung bedarfsabhängig eingestellt bzw. gesteuert werden. In besonders bevorzugter Weise erfolgt eine Regelung der Ozondosierung und/oder der Ozonherstellung auf der Basis der Messwerte. Die Prozessführungsparameter Ozon und Wasserstoffperoxid werden insbesondere am Ende des Prozesses gemessen, beispielsweise an einem Überlauf und/oder an einem Auslauf des behandelten Wassers. Ein besonderer Vorteil des erfindungsgemäßen Verfahrens ist, dass es ausreichend ist, nur ein Oxidationsmittel, insbesondere nur Ozon, in seiner Dosierung bzw. Herstellung zu steuern oder zu regeln. Das andere Oxidationsmittel, nämlich die Komponente, die durch die Ozonisierung der Olefine bereitgestellt wird, kann so eingebracht werden, dass diese Komponente latent bzw. im Überschuss im Prozess vorhanden ist.

Für den sogenannten Peroxonprozess oder auch Hydrozone-Prozess (Gleichung G3, siehe oben) ist im Allgemeinen ein bestimmtes Verhältnis von zugegebenem Ozon und Wasserstoffperoxid erforderlich, beispielsweise etwa 0,4 mg H₂O₂/ 1 Wasser für 1 mg Ozon / I Wasser. Eine optimale Dosierung von H₂O₂ hängt jedoch auch von dem Gehalt an organischen gelösten Substanzen im Wasser, also von der sogenannten Matrix ab. Dieser Gehalt ist im Allgemeinen nicht konstant, wodurch der Peroxonprozess labil ist. Der organische Substanzgehalt des Wassers, also der Gehalt an verunreinigenden Substanzen, kann zwar labortechnisch erfasst werden, ist jedoch im Allgemeinen in der Praxis nicht kontinuierlich messbar, so dass dieser Wert nicht für eine Regelung oder Steuerung des Peroxonprozesses einsetzbar ist. Wenn allein der Peroxonprozess für die oxidative Behandlung eingesetzt werden würde, könnte daher die Kettenreaktion mit den Hydroxylradikalen leicht zum Erliegen kommen, da beide Agenten, also Ozon und Wasserstoffperoxid, sich gegenseitig auslöschen können, ohne dass eine Oxidation der Schadstoffe bewirkt wird. Durch die erfindungsgemäße Behandlung mit einem Ozonid oder anderen Produkten aus der Ozonisierung von Olefinen wird jedoch eine weitere Quelle für das Hydroperoxid-Anion, das der Prekursor für die Hydroxylradikale ist, bereitgestellt, so dass es nicht zu einem Erliegen der Kettenreaktion kommt. Dabei ist es ausreichend, wenn der Prozess über die Dosierung von Ozon gesteuert oder geregelt wird.

Das erfindungsgemäße Verfahren zur Behandlung von belastetem Wasser kann in verschiedenen Bereichen mit Vorteil zum Einsatz kommen. Es ist allgemein zur Behandlung von Abwässern geeignet, wobei es beispielsweise zur Abwasserreinigung im Agrarbereich, beispielsweise zur Reinigung von Gewächshausabwässern eingesetzt werden kann, wobei insbesondere eine Oxidation und damit ein Abbau von verwendeten Pestiziden erreicht werden kann. Bei einem Einsatz in Kläranlagen können mit dem erfindungsgemäßen Verfahren beispielsweise auch pharmakologische Reststoffe abgebaut werden. Durch einen Einsatz bei der Reinigung von Schwimmbädern kann gegebenenfalls vollständig auf den Einsatz von Chlorchemie verzichtet werden. Weiterhin ist das erfindungsgemäße Verfahren auch bei einer Reinhaltung von Teichanlagen oder in der Fischzucht mit Vorteil einsetzbar.

Bei der Wasserbehandlung und vor allem bei der Abwasserbehandlung können geruchsintensive Abgase entstehen, die insbesondere durch schwefelhaltige Verbindungen verursacht werden. Das erfindungsgemäße Prinzip, wonach ein ozonisiertes Medium mit einer Komponente in Kontakt gebracht wird, die durch eine Ozonisierung von Olefinen bereitgestellt wird, lässt sich auch für eine Behandlung von Abgasen oder von Abluft einsetzen. Um die Abluft aus dem Verfahren der Abwasserbehandlung zu reinigen, kann daher in einer bevorzugten Ausgestaltung des erfindungsgemäßen Verfahrens eine oxidative Behandlung der Abluft erfolgen, indem die Abluft mit wenigstens einer Komponente in Kontakt gebracht wird, die durch die Ozonisierung von wenigstens einem Olefin bereitgestellt wird. Bei dieser Komponente handelt es sich zweckmäßigerweise um die gleiche Komponente, die auch für die eigentliche Wasserbehandlung eingesetzt wird. Bezüglich weiterer Merkmale der Komponente für die Abluftbehandlung wird daher auf die obige Beschreibung verwiesen. Vorzugsweise wird die Abluft zusätzlich ozonisiert. Es ist bevorzugt, dass die oxidativen Prozesse bei der Behandlung der Abluft in einer flüssigen Phase ablaufen. Die ablaufenden chemischen Prozesse sind dabei im Wesentlichen die Gleichen wie bei der Wasserbehandlung. Daher wird der Abluftstrom vorzugsweise befeuchtet. Dies kann mit besonderem Vorteil durch eine Gaswäsche im Abluftstrang erfolgen, die zugleich auch einen weiteren reinigenden Effekt hat.

Das erfindungsgemäße Verfahren zur Reinigung von Abgasen oder von Abluft kann auch unabhängig von der beschriebenen oxidativen Behandlung von belastetem Wasser erfolgen. Allgemein kann das Verfahren zur Behandlung von Abgasen zur Geruchsbeseitigung und zur Desinfektion in der Abluftbehandlung eingesetzt werden. Beispielsweise in der industriellen Abgasbehandlung kann das Verfahren zur Beseitigung von leichtflüchtigen organischen Substanzen (VOC) genutzt werden. Ein weiteres beispielhaftes Einsatzgebiet sind Müllsammel- und Verladestationen. Auch in der Tierzucht ist das Verfahren einsetzbar, wobei im Innenbereich hygienische Luftverhältnisse bereitgestellt werden können und außerdem eine Reinigung der Abluft durchgeführt werden kann. Bei einem Einsatz im Zusammenhang mit Klimaanlagen kann beispielsweise eine Reduzierung des Außenluftanteils als Energieeinsparungsmaßnahme erreicht werden. Mit besonderem Vorteil kann das erfindungsgemäße Verfahren im Zusammenhang mit Verdunstungskühlern eingesetzt werden, wobei insbesondere eine Reduzierung des chemischen Sauerstoffbedarfs (CSB) erzielt wird. Allgemein wird hierbei eine Desinfektion und eine Entkeimung erreicht, so dass auf chemische Desinfektionsmittel, wie z.B. Chlor, Peressigsäure, Kalumpermanganat oder Wasserstoffperoxid, verzichtet werden kann.

Weiterhin kann in effektiver Weise eine Kontamination mit Legionellen oder anderen problematischen Erregern vermieden werden. Zudem kann mit der erfindungsgemäßen Abgasbehandlung der Algenwuchs in einem Verdunstungskühler bekämpft werden.

Das erfindungsgemäße Verfahren ist ebenfalls zur oxidativen Behandlung von einer Festphase (Feststoffen) geeignet. Hierbei werden die Feststoffe einerseits mit Ozon und andererseits mit wenigstens einer Komponente, die durch die Ozonisierung von wenigstens einem Olefin bereitgestellt wird, behandelt. Da die im Zuge des erfindungsgemäßen Verfahrens ablaufenden Reaktionen in einer wässrigen Phase ablaufen, ist eine Befeuchtung der Feststoffe vorgesehen. Bezüglich weiterer Einzelheiten zu diesem Feststoffbehandlungsverfahren wird auf die obige Beschreibung verwiesen. Durch diese oxidative Behandlung kann in sehr vorteilhafter Weise eine reinigende, desinfizierende oder sterilisierende Wirkung erzielt werden. Das Verfahren kann allgemein zur Reinigung und Desinfizierung von Oberflächen eingesetzt werden, beispielsweise für eine Tankreinigung oder -konservierung oder eine Holzfassreinigung oder -konservierung, insbesondere für eine Reinigung von Weinfässern. Weiterhin eignet sich das erfindungsgemäße Verfahren zur reinigenden und desinfizierenden Aufbereitung von Feststoffabfällen, beispielsweise von Krankenhausabfällen. Krankenhausabfälle werden in der Regel verbrannt. Da aber bei Krankenhausabfällen von gesundheitsgefährdenden Kontaminationen auszugehen ist, muss vor einem Transport der Krankenhausabfälle zu einer Müllverbrennungsanlage in der Regel eine Dekontamination der Abfälle durchgeführt werden. Das erfindungsgemäße Verfahren eignet sich für die Behandlung von Krankenhausabfällen in besonderem Maße, da die Ozonidsubstanz, mit der die Feststoffe behandelt werden, den Feststoffen zumindest für eine gewisse Zeit anhaftet und auch noch bei einem Transport ihre oxidative Wirkung entfaltet. Die erfindungsgemäße Behandlung übt gewissermaßen eine Depotwirkung aus, so dass eine längerfristige Dekontamination erreicht wird und beispielsweise eine erneute Vermehrung von Krankheitserregern während eines Transportes der Abfälle vermieden wird.

Vorzugsweise werden die Abfälle vor der erfindungsgemäßen oxidativen Behandlung oder gegebenenfalls auch währenddessen zerkleinert, beispielsweise geschreddert oder gemahlen. Durch die hiermit verbundene Oberflächenvergrößerung der Feststoffe kann die erfindungsgemäße Behandlung besonders effektiv Ihre Wirkung entfalten.

Zweckmäßigerweise werden die Feststoffe vor oder während der oxidativen Behandlung befeuchtet. Insbesondere kann die Befeuchtung mit ozonisiertem Wasser erfolgen, beispielsweise durch Besprühen oder Beregnen, so dass die Befeuchtung und die Behandlung mit Ozon in einem Schritt durchgeführt werden.

Das erfindungsgemäße Verfahren kann weiterhin mit Vorteil im Pflanzenschutz und insbesondere bei der Behandlung von Pflanzenschädlingen eingesetzt werden. Versuche des Erfinders haben gezeigt, dass durch eine Behandlung mit Ozon und mit wenigstens einer Komponente, die durch die Ozonisierung eines Olefins bereitgestellt wird, ein Pilzbefall oder ein Insektenbefall wirksam bekämpft werden kann.

Nachfolgend wird eine Vorrichtung zur oxidativen Behandlung von einer Flüssigphase und/oder einer Gasphase und/oder Festphase beschrieben. Diese umfasst wenigstens eine Einrichtung zur Einbringung von Ozon und wenigstens eine Einrichtung zur Einbringung einer Komponente, die durch die Ozonisierung von wenigstens einem Olefin bereitgestellt wird, in den Prozess. Bezüglich weiterer Merkmale der einzelnen Elemente der Vorrichtung wird auch auf die vorhergehende Beschreibung verwiesen.

Vorzugsweise enthält die Vorrichtung eine Packung, die zur Adsorption der Komponente, die durch die Ozonisierung des wenigstens einen Olefins bereitgestellt wird, vorgesehen ist.

Wenn in der Vorrichtung ein Abluftstrang vorhanden ist, können auch im Bereich des Abluftstrangs eine (weitere) Einrichtung zur Einbringung einer Ozonidkomponente und/oder eine Einrichtung zur Einbringung von Ozon und/oder eine Einrichtung zur Befeuchtung des Luftstroms, z.B. ein Gaswäscher, vorgesehen sein. Durch diese Maßnahme(n) kann auch die Abluft, unabhängig von beispielsweise einer Flüssigphase oder einer Festphase, die in der Vorrichtung behandelt wird, zusätzlich in der erfindungsgemäßen Weise oxidativ behandelt werden.

Die beschriebene Vorrichtung ist insbesondere zur oxidativen Behandlung von belastetem Wasser, beispielsweise von Abwässern, geeignet. In einem ersten Schritt wird gasförmiges Ozon in das Wasser eingebracht, beispielsweise durch einen entsprechenden Injektor. Das Ozon wird im Wasser physikalisch gelöst. Nachfolgend wird das ozonierte Wasser mit der Komponente, die durch die Ozonisierung der Olefine bereitgestellt wird, in Kontakt gebracht. Diese Reihenfolge von Ozonisierung und Zugabe dieser Komponente ist jedoch nicht zwingend und kann auch in anderer Weise erfolgen. Das im Wasser gelöste Ozon wird letztendlich zu Hydroxylradikalen transformiert. Hierbei wird eine radikalische Kettenreaktion ausgelöst, die die Oxidation der im Wasser enthaltenen Schadstoffe bewirkt, so dass die Schadstoffe abgebaut und mineralisiert werden. Weiterhin wird durch die oxidative Behandlung eine Desinfektion oder Entkeimung des Wassers erreicht.

Für die Reaktion sind ein oder mehrere Reaktionsbecken vorgesehen. Vorzugsweise findet in dem oder den Reaktionsbecken eine Zirkulation statt, so dass das Wasser solange im Reaktionsbecken gehalten wird kann, bis der Prozess abgeschlossen ist, wobei vorzugsweise eine kontinuierliche Prozessführung vorgesehen ist.

In einer bevorzugten Ausgestaltung der beschriebenen Vorrichtung umfasst die Vorrichtung wenigstens einen Rohrreaktor, der in dem Reaktionsbecken der Vorrichtung vorgesehen ist. Das zu behandelnde Wasser wird beispielsweise mittels einer Pumpe durch diesen Rohrreaktor geführt. Durch die Strömungsverhältnisse im und stromabwärts des Rohrreaktors wird eine Zirkulation des Wassers erreicht. Die Komponente, die durch die Ozonisierung der Olefine bereitgestellt wird, bzw. die Ozonidsubstanz kann direkt in den Rohrreaktor eingebracht werden. Im Rohrreaktor kann eine Packung beispielsweise aus Kieselsteinen oder anderen Materialen vorgesehen sein, an die das Ozonid bzw. die Komponente adsorbiert. Eine solche Packung kann jedoch auch alternativ oder zusätzlich an anderer Stelle in einem Becken der Vorrichtung bzw. der Anlage vorgesehen sein. Auch die Dosierstelle für das Ozongas kann in den Rohrreaktor münden, vorzugsweise stromaufwärts der gegebenenfalls vorhandenen Dosierstelle für die Ozonidsubstanz. In anderen Ausgestaltungen kann es auch vorgesehen sein, dass die Ozonierung des Wassers an anderer Stelle erfolgt.

Die Ozonierung des Wassers, also die Lösung von gasförmigem Ozon in der Wasserphase, kann beispielsweise im Rohrreaktor und/oder an anderer Stelle im System erfolgen. Das Ozon wird vorzugsweise über eine oder mehrere Ozondosierstellen eingebracht, die beispielsweise mit einem Feinfilter in Form einer Fritte und/oder als Ozoninjektor ausgestaltet sind. Da die nachfolgenden oxidativen Prozesse in der Regel sehr schnell verlaufen, kann die Lösung des Ozons im Wasser der geschwindigkeitsbestimmende Schritt des Verfahrens sein. Um den Zeitablauf des Prozesses weiter zu optimieren, können Einrichtungen zur verbesserten Gaslösung des Ozons im Wasser vorgesehen sein. Beispielsweise kann ein Ozoninjektor verwendet werden, der nach dem Prinzip einer Venturi-Düse die Vermischung der Gasphase und der Wasserphase im Bereich der Dosierstelle verstärkt und damit die Gaslösung verbessert. Zusätzlich oder alternativ kann stromabwärts der Ozondosierstelle eine turbulente Rohrstrecke vorgesehen sein, die ebenfalls die Lösung des Ozongases in der Wasserphase verstärkt. Eine derartige turbulente Rohrstrecke kann beispielsweise mindestens 10 Meter in der Länge betragen, wobei Strukturen in der Rohrstrecke vorgesehen sein können, die eine hohe Reynoldszahl (Re), zum Beispiel Re > 5000, als Maß für das Turbulenzverhalten bewirken.

Die in einer bevorzugten Ausgestaltung der Vorrichtung vorgesehene Packung stellt Oberflächen zur Adsorption der Ozonidsubstanz bereit. Geeignet hierfür sind beispielsweise Kieselsteine und/oder Materialien aus Metalloxiden und/oder aus Keramik und/oder Glas. Diese Materialien können beispielsweise in Form einer Packung in dem Rohrreaktor enthalten sein, vorzugsweise stromabwärts einer Dosierstelle für das Ozongas, und/oder an anderer Stelle im System. Diese Materialien bzw. Strukturen weisen mit besonderem Vorteil eine oxidische Oberfläche auf. Die Strukturen bzw. die Packung dienen/dient damit zum einen als adsorptiver Träger für die Ozonidsubstanz. Zum anderen kann an den oxidischen Oberflächen ebenfalls Ozon umgesetzt werden, so dass diese Oberflächen eine weitere Quelle für die Bildung der Hydroxylradikale darstellen.

In einer weiteren bevorzugten Ausgestaltung der beschriebenen Vorrichtung umfasst die Vorrichtung wenigstens eine UV-Strahlungsquelle, beispielsweise einen oder mehrere UV-Strahler (Wellenlänge z.B. 254 nm). Durch die UV-Bestrahlung wird das im Wasser enthaltene Wasserstoffperoxid zu Hydroxylradikalen umgesetzt. Somit wird durch einen oder mehrere UV-Strahler beispielsweise in einem oder mehreren Reaktionsbecken eine weitere Quelle für die Hydroxylradikal-Bildung bereitgestellt, die den oxidativen Aufreinigungsprozess weiter verbessert. Zudem kann durch eine UV-Bestrahlung überschüssiges Wasserstoffperoxid im Wasser abgebaut werden. Dies kommt insbesondere dann zum Tragen, wenn die Abbauprozesse abgeschlossen sind und dem Wasserstoffperoxid weitere Reaktionsmöglichkeiten fehlen. Um das Wasser von überschüssigem Wasserstoffperoxid zu befreien, kann eine UV-Strahlungsquelle insbesondere im Bereich eines Auslaufs der Vorrichtung angeordnet sein. Es können auch mehrere UV-Strahlungsquellen an unterschiedlichen Positionen in der Vorrichtung oder Anlage bzw. an unterschiedlichen Prozessstufen vorgesehen sein.

Zweckmäßigerweise umfasst die beschriebene Vorrichtung weiterhin wenigstens eine Einrichtung zur Messung der Ozonkonzentration und/oder der Wasserstoffperoxidkonzentration. Diese Prozessführungsparameter können insbesondere kontinuierlich gemessen werden. Auf der Basis der gemessenen Werte kann die Prozessführung über eine geeignete Steuerungs- oder Regelungseinheit vorgenommen werden. Die Messung dieser Parameter erfolgt vorzugsweise am Ende des Prozesses, beispielsweise an einem Überlauf und/oder an einem Auslauf des behandelten Wassers. Beispielsweise können die Messungen, insbesondere die Wasserstoffperoxidmessung, an einem Überlauf für das behandelte Wasser erfolgen, bevor das Wasser in ein Becken läuft, in dem eine UV-Bestrahlung stattfindet. Die UV-Bestrahlung kann in Abhängigkeit von der gemessenen Wasserstoffperoxidkonzentration erfolgen. Aus dem UV-Bestrahlungsbecken läuft das Wasser in eine Abflussrinne für das behandelte Wasser (Reinstwasserrinne). An dieser Position kann eine nochmalige Messung der Parameter vorgesehen sein.

Die Messung des im Wasser gelösten Ozons kann mit verschiedenen Methoden erfolgen. Besonders bevorzugt ist ein Messverfahren, bei dem die Strahlungsabsorption des Gases gemessen und mit einer Referenzabsorption verglichen wird, wobei die Lösungen in je eine Küvette geleitet und beide Küvetten abwechselnd vermessen werden. Ein solches Verfahren geht beispielsweise aus der deutschen Patentschrift DE 41 19 346 C2 hervor. Dieses Verfahren ist besonders geeignet, da auch in Gegenwart von organischen Substanzen sowie von peroxidischen Substanzen das im Wasser gelöste Ozon zuverlässig bestimmt werden kann. Für die Messung des Wasserstoffperoxids kann mit besonderem Vorteil ein Amperometer eingesetzt werden, das insbesondere auf der Basis von drei Elektroden und einen Potentiostat arbeitet.

Die oxidative Wasserbehandlung läuft mit besonderem Vorteil im basischen Milieu ab. Zur Einstellung eines optimalen pH-Wertes umfasst die erfindungsgemäße Vorrichtung eine Einrichtung zur Messung und gegebenenfalls zur Steuerung und/oder Regelung des pH-Wertes. Vorzugsweise sind hierbei Mittel zur Einstellung eines basischen pH-Wertes vorgesehen. Besonders vorteilhaft ist beispielsweise ein pH-Wert von 8 oder größer. In diesem pH-Bereich findet die Bildung der für die oxidative Behandlung erforderlichen Hydroxylradikale in besonders starkem Maße statt. Weiterhin entweicht das bei den Abbaureaktionen gebildete Kohlendioxid in diesem pH-Bereich in gasförmiger Form nahezu vollständig aus dem Wasser. Untersuchungen des Erfinders haben beispielsweise gezeigt, dass bei einem pH-Wert von 7,9 im Wasser nach Durchlaufen des Prozesses Ozon und Wasserstoffperoxid noch gemessen werden können, bei einem pH-Wert von 8,4 hingegen nicht mehr. Eine pH-Messung kann beispielsweise gleich zu Beginn des Prozesses am Einlauf des zu behandelnden Wassers in das Reaktionsbecken erfolgen.

Die Vorrichtung kann weiterhin einen Ozonreduktor, der zur Beseitigung von überschüssigem Ozon insbesondere aus der Luft nach Durchlaufen des Prozesses vorgesehen ist, umfassen. Der Ozonreduktor kann insbesondere für eine thermische Zerstörung des Ozons vorgesehen sein. Beispielsweise kann hierfür eine an sich bekannte Vorrichtung zur Zerstörung von Ozon aus Gasen mit integriertem Wärmetauscher eingesetzt werden, wie sie insbesondere aus der deutschen Patentschrift DE 10 2004 051 945 B4 bekannt ist. Vorzugsweise ist die Dosierstelle für das Ozongas stromaufwärts der Einrichtung zur Einbringung der Ozonidsubstanz angeordnet. Vorteilhaft für die Durchführung des Prozesses ist, dass das Ozongas im zu behandelnden Wasser bereits gelöst ist, bevor das Wasser in Kontakt mit der Ozonidsubstanz kommt. Insbesondere wenn das Wasser in einem Reaktionsbecken zirkuliert und/oder die Ozonidsubstanz in adsorbierter Form vorliegt, können die Dosierstelle für das Ozongas und die Dosierstelle für die Ozonidsubstanz auch anders angeordnet sein.

Die beschriebene Vorrichtung oder Anlage kann mit einer Abluftbehandlung kombiniert werden, so dass störende und/oder schädliche Geruchsemissionen, die bei der Wasserbehandlung entstehen können, beseitigt werden können. Derartige Geruchsemissionen können insbesondere durch die Bildung von Schwefelverbindungen, insbesondere Schwefelwasserstoff und Mercaptane (Thioalkohole), oder allgemein durch bakterielle Abbauprodukte entstehen, die in die Gasphase übertreten.

In besonders bevorzugter Weise erfolgt eine oxidative Behandlung der Abluft oder der Abgase aus der Wasserbehandlung. Hierbei werden im Prinzip die gleichen chemischen Prozesse genutzt, die auch bei der Abwasserbehandlung eingesetzt werden, wobei eine Behandlung der Abluft mit einer Komponente erfolgt, die durch die Ozonisierung von wenigstens einem Olefin bereitgestellt wird (Ozonidsubstanz). Insbesondere in Gegenwart von Ozon in der Abluft werden hierdurch oxidative Prozesse ausgelöst, die zu einer Eliminierung von Verunreinigungen in der Abluft und vor allem zum Abbau von schwefelhaltigen Verbindungen zu Schwefeldioxid führen. Die beschriebene Vorrichtung weist daher vorteilhafterweise eine Einrichtung zur Einbringung dieser Komponente in den Abluftstrang auf, insbesondere eine Dosierstelle für die Ozonidsubstanz. Weiterhin ist vorteilhafterweise eine Einrichtung zur Einbringung von Ozon in den Abluftstrang vorgesehen. Zu diesem Zweck kann auch ozonhaltige Abluft aus dem System eingesetzt werden.

In einer bevorzugten Ausführungsform ist weiterhin eine Einrichtung zur Befeuchtung des Luftstroms vorgesehen. Hierdurch kann die Prozessführung bei der Abluftbehandlung so gestaltet werden, dass die oxidativen Prozesse in einer wässrigen bzw. nassen Phase ablaufen können. Als Einrichtung zur Befeuchtung eignen sich in besonderer Weise ein oder mehrere Gaswäscher. Bei einem Gaswäscher werden in an sich bekannter Weise Bestandteile eines Gasstroms über einen Waschflüssigkeitsstrom ausgewaschen. Auf diese Weise kann auf der einen Seite eine Befeuchtung des Luftstroms erreicht werden. Auf der anderen Seite wird ein weiterer reinigender Effekt erreicht. Der oder die Gaswäscher können vorzugsweise mit dem Wasser aus dem oder den Reaktionsbecken betrieben werden. Der Waschflüssigkeitsstrom und Kondensate aus dem Gaswäscher können in das oder die Reaktionsbecken der Abwasserbehandlung zurückgeführt oder anderweitig abgeleitet werden. In dieser Ausgestaltung der Erfindung kann das Wasser zum Einen durch die oxidative Behandlung desinfiziert und von Schadstoffen befreit werden. Zugleich können zum Anderen geruchsintensive Verbindungen, die bei der Abwasserreinigung entstehen, durch eine Gaswäsche und gegebenenfalls vor allem auch durch die oxidative Behandlung der Abluft entfernt werden. Die beschriebene Vorrichtung kann daher mit besonderem Vorteil beispielsweise auch als Bestandteil einer Kläranlage eingesetzt werden.

Für die Behandlung der Abluft mit der Komponente, die durch die Ozonisierung der Olefine bereitgestellt wird (Ozonidsubstanz), ist vorzugsweise eine Packung, z.B. eine Packung mit Kieselsteinen, vorgesehen, an die die Ozonidsubstanz adsorbiert. Diese Packung wird von der Abluft passiert. Eine Ozonzugabe kann über den Ozongenerator der Wasserbehandlungsanlage erfolgen. Auf eine Ozonzugabe von außen kann unter Umständen verzichtet werden, wenn ozonhaltige Abluft aus dem System eingesetzt wird. In der Anlage tritt bei entsprechender Dosierung von Ozon während der Wasserbehandlung überschüssiges Ozon in die Gasphase über, das für diesen Zweck eingesetzt werden kann. Durch die Wiederverwertung dieser ozonhaltigen Abluft aus der Anlage für die oxidativen Prozesse bei der Abluftbehandlung kann gegebenenfalls auf den oben beschriebenen Ozonzerstörer bei der Abluftbehandlung verzichtet werden. Als oxidierende Substanz bei der Abluftbehandlung kann zusätzlich oder alternativ auch Wasserstoffperoxid wirken, das aus dem Wasser aus den Reaktionsbecken der Anlage stammen kann, wenn der oder die Gaswäscher mit diesem Wasser betrieben werden. In besonders vorteilhafter Weise kann daher die oxidative Behandlung der Abluft mit einem Gaswäscher kombiniert werden, der mit dem Wasserstoffperoxid-haltigen Wasser aus dem oder den Reaktionsbecken betrieben wird. Eine alternative oder zusätzliche Quelle für Wasserstoffperoxid kann die Packung mit der adsorbierten Ozonidsubstanz im Abgasstrang bilden. Es ist also nicht erforderlich, externes Wasserstoffperoxid zuzugeben. Die oxidativen Prozesse in der Abluft laufen bevorzugt in einem basischen Milieu ab. Da das Wasser aus dem oder den Reaktionsbecken in bevorzugter Weise bereits einen basischen pH-Wert aufweist, ist eine Zugabe von potentiell umweltschädlichen basischen Reagenzien zur Einstellung des pH-Wertes, wie beispielsweise Natronlauge, nicht erforderlich.

Durch die ausgelösten oxidativen Prozesse in der Abluft können beispielsweise die Thioalkohol-Verbindungen und Schwefelwasserstoff in der Abluft zu Schwefeldioxid oxidiert werden. Schwefeldioxid ist wasserlöslich und kann mit einem gegebenenfalls zusätzlichen Gaswäscher ausgewaschen werden. Es kann beispielsweise in Fließrichtung der Abluft ein erster Gaswäscher, dann eine Packung zur Absorption der Ozonidsubstanz und anschließend ein weiterer Gaswäscher vorgesehen sein. Stromaufwärts der Packung kann die Ozonidsubstanz in flüssiger Form oder gas- oder dampfförmig eindosiert werden. Wenn gasförmiges Ozonid eingesetzt wird, kann hierfür Ozonidgas verwendet werden, das bei der Herstellung der pastösen Ozonidsubstanz für die Wasserbehandlung entsteht. Es sind auch Ausführungsformen mit nur einem Gaswäscher möglich, der stromaufwärts oder stromabwärts einer gegebenenfalls vorhandenen Packung zur Absorption der Ozonidsubstanz angeordnet ist. Insbesondere bei hohen Schwefelwasserstoffkonzentrationen in der Abluft, beispielsweise bei 1000 ppm oder mehr, sind zwei Gaswäscher vorteilhaft, die stromaufwärts bzw. stromabwärts der Packung für die Adsorption der Ozonidsubstanz angeordnet sind. Das saure Kondensat aus dem oder den Gaswäscher(n) kann zur Einstellung des pH-Wertes im Auslauf oder gegebenenfalls zusätzlich oder alternativ im Einlauf der Wasserbehandlungsanlage verwendet werden.

Besonders hohe Schwefelwasserstoffkonzentrationen entstehen beispielsweise bei einem Ausfaulen der Klärschlämme. Zum oxidativen Abbau der geruchsintensiven Verbindungen der hierbei entstehenden Abgase sind hohe Oxididationskapazitäten erforderlich, die insbesondere durch die erfindungsgemäße Kombination einer Ozonzugabe mit der weiteren oxidierenden Komponente (Ozonidsubstanz oder Ozonid) vorzugsweise in Kombination mit einer Gaswäsche erreicht werden. Eine Regelung der Ozonzugabe kann beispielsweise durch eine Messung der Schwefelwasserstoffkonzentration am Abgasausgang erfolgen.

Diese Abgasbehandlungsvorrichtung ist nicht auf solche Anlagen beschränkt, die eine Wasserbehandlung auf der Basis von Ozon und einer Ozonidsubstanz gemäß der obigen Beschreibung durchführen. Vielmehr kann diese oxidative Abgasbehandlung auch für andere Abgase oder Abluft eingesetzt werden, wobei die Einrichtung zur Einbringung von Ozon eine Einrichtung zur Ozonisierung der Abgase oder der Abluft ist. Die Erfindung umfasst daher auch eine Vorrichtung zur Reinigung von Abgasen unabhängig von der erfindungsgemäßen Abwasserbehandlung. Insbesondere eignet sich diese Abgas- oder Abluftbehandlung allgemein für die Abluftbehandlung in Kläranlagen. Mit der Vorrichtung können vor allem schwefelhaltige Verbindungen als geruchsintensive Emissionen aus der Luft (Abluft) entfernt werden. Die oxidativen und reinigenden Prozesse in der Abluft werden durch eine Behandlung mit einer oxidierenden Komponente, die durch die Ozonisierung von wenigstens einem Olefin bereitgestellt wird, insbesondere in Gegenwart von Ozon als weiterem Oxidationsmittel ausgelöst. Die Komponente auf der Basis von ozonisierten Olefinen liegt vorzugsweise in adsorbierter Form an einer Packung vor, die von der Abluft passiert wird. Die Packung kann beispielsweise aus Kieselsteinen oder anderen Materialien bestehen. Die hierdurch ausgelösten chemischen Prozesse entsprechen im Wesentlichen den Prozessen, die auch bei der erfindungsgemäßen Wasserbehandlung ablaufen. Insofern wird bezüglich weiterer Einzelheiten insbesondere im Hinblick auf die oxidierende Komponente auf die obige Beschreibung zur Abwasserbehandlung verwiesen.

Die Prozessführung kann so ausgestaltet sein, dass Ozon in den Abluftstrang eindosiert wird. Es kann gegebenenfalls auch ozonhaltige Abluft aus einer Gesamtanlage hierfür verwendet werden. Die Komponente auf der Basis der ozonisierten Olefine kann in flüssiger oder gasförmiger Form dosiert werden. Die oxidativen Prozesse können weiterhin durch die Gegenwart von Wasserstoffperoxid verstärkt werden. Vorzugsweise wird Wasserstoffperoxid durch Wasserstoffperoxid-haltige Flüssigkeiten, insbesondere aus Wasser, die bzw. das aus dem Gesamtsystem stammt, bereitgestellt. Mit besonderem Vorteil ist wenigstens eine Einrichtung zur Befeuchtung des Luftstroms vorgesehen, wobei hierfür insbesondere ein oder mehrere Gaswäscher eingesetzt werden können. Für die Gaswäsche kann Wasser, insbesondere Wasserstoffperoxid-haltiges Wasser, aus dem Gesamtsystem, als Waschflüssigkeit eingesetzt werden. Im Prinzip basiert die erfindungsgemäße Abluftbehandlung darauf, dass feuchtes Ozongas über eine Ozonidoberfläche oder eine andere peroxidische Oberfläche geleitet wird, wobei die Befeuchtung des Ozongases vorzugsweise mittels eines Gaswäschers erreicht wird.

Die beschriebene Vorrichtung ist weiterhin zur oxidativen Behandlung von Feststoffen geeignet, beispielsweise zur Behandlung von Fässern, beispielsweise Holzfässern, oder von Tanks oder zur Behandlung von Abfällen. Für die Einbringung von Ozon und für die Einbringung der Ozonidsubstanz können eine gemeinsame Einrichtung oder separate Einrichtungen, beispielsweise zwei verschiedene Dosierstellen, vorgesehen sein. Weiterhin kann eine Befeuchtungseinrichtung, beispielsweise eine Beregnungseinrichtung, vorgesehen sein, mit der Wasser, das gegebenenfalls ozonisiert sein kann, eingebracht wird. Diese Beregnungseinrichtung kann in diesem Fall auch die Einrichtung zur Einbringung von Ozon darstellen. Je nach Art der zu behandelnden Feststoffe oder Oberflächen kann weiterhin eine Zerkleinerungseinrichtung vorgesehen sein, beispielsweise für die Behandlung von Müll. Bei der Behandlung von Fässern oder Tanks mit einer solchen Vorrichtung wird vor allem eine Desinfektion der inneren Oberflächen der Fässer oder Tanks erreicht. Bei einer Vorrichtung zur Behandlung von Fässern oder Tanks kann beispielsweise ein Sprühkopf, der zweckmäßigerweise an der Eintrittsstelle in das Fass oder den Tank abgedichtet wird, im Inneren des Fasses oder des Tanks angeordnet werden. Über den Sprühkopf kann die Einbringung von Ozon und von der Ozonidsubstanz gleichzeitig oder zeitlich versetzt erfolgen. Weiterhin kann eine Absaugvorrichtung zur abschließenden Entfernung von Flüssigkeiten aus dem Fass oder dem Tank vorgesehen sein.

Beschrieben wird weiterhin ein Verdunstungskühler, dem wenigstens eine Einrichtung zur Einbringung von Ozon in den Verdunstungskühler und zur Einbringung einer Komponente, die durch die Ozonisierung von wenigstens einem Olefin bereitgestellt wird (Ozonidsubstanz), in den Verdunstungskühler, zugeordnet ist. Für die Einbringung von Ozon und für die Einbringung der Ozonidsubstanz können eine gemeinsame Einrichtung oder separate Einrichtungen, beispielsweise zwei verschiedene Dosierstellen, vorgesehen sein. Eine Verkeimung bei Verdunstungskühlern, die zum Beispiel im Rahmen von Klimaanlagen eingesetzt werden, ist ein weit verbreitetes Problem. Insbesondere wenn in dem Verdunstungskühler eine Packung zur Vergrößerung der Oberfläche verwendet wird, ist die Gefahr von Kontaminationen groß. Der Einsatz des oben beschriebenen Verfahrens für derartige Verdunstungskühler, also die kombinierte Behandlung des Verdunstungskühlers mit Ozon und mit der Ozonidsubstanz, hat den Vorteil, dass in effektiver Weise eine Hygienisierung des Verdunstungskühlers durch die ablaufenden oxidativen Prozesse erreicht wird, wobei eine Kontamination mit beispielsweise Algen und Bakterien sicher vermieden wird. Gleichzeitig sind die ausgelösten oxidativen Kettenreaktionen so wirkungsvoll, dass das eingesetzte Ozon vollständig umgesetzt wird und es daher nicht zu potentiell schädlichen Ozonemissionen kommt.

Weitere Merkmale und Vorteile der Erfindung ergeben sich aus der nachfolgenden Beschreibung von Ausführungsbeispielen im Zusammenhang mit den Zeichnungen. Hierbei können die einzelnen Merkmale jeweils für sich oder in Kombination miteinander verwirklicht sein.

In den Figuren zeigen:
Figur 1 eine erste Ausführungsform einer Vorrichtung zur oxidativen Behandlung von belastetem Wasser;
Figur 2 eine zweite Ausführungsform einer Vorrichtung zur oxidativen Behandlung von belastetem Wasser;
Figur 3 eine dritte Ausführungsform einer Vorrichtung zur oxidativen Behandlung von belastetem Wasser;
Figur 4 eine vierte Ausführungsform einer Vorrichtung zur oxidativen Behandlung von belastetem Wasser mit zusätzlicher Abluftbehandlung;
Figur 5 eine Ausführungsform einer Vorrichtung zur oxidativen Behandlung von zerkleinerten Feststoffen;
Figur 6 eine Ausführungsform einer Vorrichtung zur oxidativen Behandlung von Fässern oder Tanks und
Figur 7 einen Verdunstungskühlers.

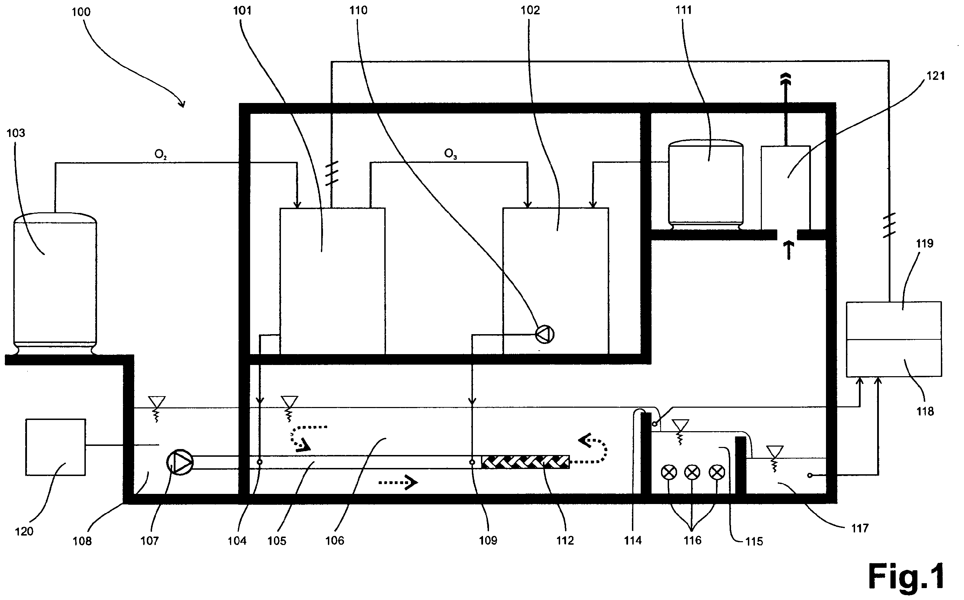

Figur 1 illustriert ein erstes Ausführungsbeispiel für eine geeignete Vorrichtung 100 zur oxidativen Behandlung von belastetem Wasser. Wesentliche Bestandteile der Vorrichtung sind ein Ozongenerator 101 und eine Einheit 102, mit der eine Ozonidsubstanz, insbesondere organische Ozonide (und/oder Peroxide und/oder Hydroperoxide), bereitgestellt werden. In einem Tank 103 wird trockener Sauerstoff vorgehalten, der in den Ozongenerator 101 eingeleitet wird. Der Sauerstoff dient als Trägergas für die Ozonbildung im Ozongenerator 101, wobei das Ozon insbesondere elektrisch durch Plasmaentladung in an sich bekannter Weise hergestellt wird. Das gebildete Ozon wird über eine Ozondosierstelle 104, die vorzugsweise mit einem Feinfilter (Fritte) ausgestattet ist, in einen Rohrreaktor 105 eingeleitet. Der Rohrreaktor 105 befindet sich in einem Reaktionsbecken 106. Das zu behandelnde Wasser wird mittels einer Pumpe 107 in Wassereinlaufbecken 108 gefördert und in den Rohrreaktor 105 eingeleitet. Das zu behandelnde Wasser durchläuft den Rohrreaktor 105 und wird im Bereich der Dosierstelle 104 mit Ozon versetzt. Das gasförmige Ozon löst sich in der Wasserphase. Im Rohrreaktor 105 herrschen vorzugsweise turbulente Strömungsverhältnisse, die eine effiziente Gaslösung fördern. Das Ozon kann dabei direkt beispielsweise mit aliphatischen Schadstoffen sowie mit bakteriellen Substanzen, insbesondere mit Proteinen und Lipiden, reagieren, und eine erste oxidative Behandlungsstufe realisieren. Im weiteren Verlauf des Rohrreaktors 105 befindet sich eine Dosierstelle 109 für das organische Ozonid bzw. die Ozonidsubstanz. Das Ozonid gelangt über eine Pumpe 110, beispielsweise eine Zahnradpumpe, zu der Dosierstelle 109 und wird beispielsweise tröpfchenweise zugegeben.

Ausgangsstoff für die Ozonidherstellung in der Einheit 102 ist in diesem Beispiel eine einfach oder mehrfach ungesättigte Fettsäure, insbesondere Ölsäure, die in Form von nativem Olivenöl im Vorratsbehälter 111 bereitgehalten wird. Das Ozonid bildet sich in der Einheit 102 aprotisch gemäß dem Criegee-Mechanismus, wobei trockenes Ozongas aus dem Ozongenerator 101 als Trägergas dient. Das Ozonid (ozonisiertes Olivenöl) bildet eine pastöse oder cremeartige Substanz, die mittels der Zahnradpumpe 110 in den Rohrreaktor 105 eingebracht wird. In anderen Ausgestaltungen kann es vorgesehen sein, dass das Ozonid an anderem Ort hergestellt wird und als fertige Ozonidsubstanz zur Anlage transportiert und dort eingesetzt wird.

Für die Ozonidherstellung wird das Ozongas in die zu ozonisierende Substanz, also beispielsweise in das native Olivenöl, eingeleitet. Die Konzentration des Ozongases kann dabei beispielsweise 120 g O₃ / m³ O₂ betragen. Je nach Auslegung der Anlage kann die Ozonidherstellung nach circa 2 bis 8 Stunden abgeschlossen sein. Der erfolgreiche Ozonisierungsprozess lässt sich insbesondere anhand der Konsistenz und der Färbung der resultierenden Masse beobachten, die im Zuge des Ozonisierungsprozesses zunehmend pastöser und weißlicher wird. Das resultierende Ozonid weist einen sehr hohen Gehalt an peroxidischen Verbindungen auf. Die Peroxidzahl kann beispielsweise zwischen etwa 300 und 1000 oder höher liegen, insbesondere bis zu 6000.

Stromabwärts der Dosierstelle 109 befindet sich im Rohrreaktor 105 ein Bereich 112, der eine Packung enthält, an die die nahezu nicht wasserlösliche Ozonidsubstanz adsorbiert. Hierbei handelt es sich vorzugsweise um Kieselsteine oder andere Strukturen, beispielsweise aus metalloxidischen oder keramischen Materialien oder aus Glas, die für eine Adsorption der Ozonidsubstanz geeignet sind. Durch die Adsorption der Ozonidsubstanz stehen immer Spuren von Ozonid oder anderen peroxidischen Verbindungen für die oxidativen Reaktionen zur Verfügung. Mit besonderem Vorteil weisen die Adsorptionsmaterialien oxidische Oberflächen auf, an denen eine weitere Umsetzung des im Wasser gelösten Ozons erfolgt. Metalloxide oder Keramiken eignen sich insbesondere für eine Prozessführung bei pH-Werten oberhalb von 7, beispielsweise kann Aluminiumoxid oder Manganoxid eingesetzt werden. Weitere geeignete Materialien sind beispielsweise Siliziumoxid (Silicagel) oder poröses Glas.

Die hohe Ausströmungsgeschwindigkeit aus dem Rohrreaktor 105 führt zu einer Zirkulationsbewegung im Reaktionsbecken 106, hier angedeutet durch gestrichelte Pfeile. Durch die Förderleistung der Pumpe 107 entsteht im Bereich 114 ein Überlauf, wobei das Wasser in ein Überlaufbecken 115 geleitet wird. In dem Überlaufbecken 115 ist eine Einrichtung 116 zur UV-Bestrahlung des Wassers vorgesehen. In diesem Bereich wird überschüssiges Wasserstoffperoxid durch die UV-Bestrahlung zerstört. Das überlaufende Reinstwasser gelangt in die Reinstwasserrinne 117 als Auslauf der Vorrichtung 100. Das Wasser im Bereich der Reinstwasserrinne 117 wird kontinuierlich vermessen im Hinblick auf die Ozonkonzentration und die Wasserstoffperoxidkonzentration. Hierfür sind eine Messeinrichtung 118 für Wasserstoffperoxid und eine Messeinrichtung 119 für Ozon vorgesehen. Eine weitere Wasserstoffperoxidmessung kann insbesondere auch im Bereich des Überlaufs 114 zwischen dem Reaktionsbecken 106 und dem Überlaufbecken 15 erfolgen. Auf der Basis der mit diesen Messungen erfassten Prozessparameter kann eine Steuerung und/oder Regelung der Ozondosierung und/oder Ozonproduktion im Ozongenerator 101 erfolgen. Hierfür ist eine nicht näher dargestellte Steuerungs- und/oder Regelungseinheit vorgesehen, so dass die Ozonproduktion und/oder die Ozondosierung verbrauchsabhängig geregelt oder gesteuert werden können. Überschüssiges Ozongas, das aus dem Wasser austritt, kann über den Ozonzerstörer 121, der oberhalb der Reinstwasserrinne 17 angeordnet ist, thermisch elimiert werden.

Im Bereich des Wassereinlaufs 108 ist eine pH-Wert-Messeinheit 120 vorgesehen. Hiermit wird der pH-Wert des eingeleiteten Wassers erfasst. Da das erfindungsgemäße Verfahren vorzugsweise im basischen Bereich, insbesondere bei einem pH-Wert von 7 oder größer, insbesondere bei pH 8 oder größer, durchgeführt wird, wird der pH-Wert gegebenenfalls auf einen geeigneten pH-Wert eingestellt.

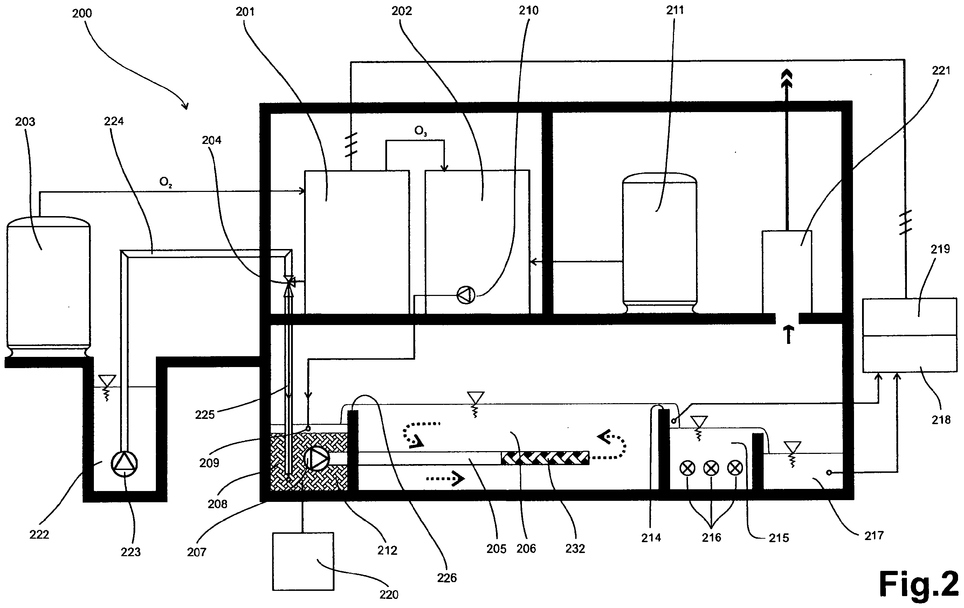

Figur 2 illustriert eine weitere Ausführungsform einer Vorrichtung 200 zur oxidativen Wasserbehandlung. Vergleichbar mit der Ausführungsform aus Figur 1 ist ein Ozongenerator 201 und eine Einheit 202 für die Bereitstellung der Ozonidsubstanz vorgesehen. Als Trägergas für die Ozonherstellung in dem Ozongenerator 201 wird trockener Sauerstoff eingesetzt, der in dem Behältnis 203 vorgehalten wird. Eine Ozonisierung von ungesättigten Fettsäuren in der Einheit 202 erfolgt auch in diesem Beispiel unter Verwendung von Ölsäure, das in dem Vorratsbehältnis 211 in Form von nativem Olivenöl bereitgehalten wird.

In diesem Ausführungsbeispiel liegt ein besonderer Schwerpunkt der Wasserbehandlung bei der direkten Ozonreaktion, die insbesondere auch eine Desinfektion oder Entkeimung des zu behandelnden Wassers bewirkt. Dieses Ausführungsbeispiel unterscheidet sich daher von dem in Figur 1 illustrierten Beispiel durch die Maßnahmen zur Einbringung des Ozongases in das Wasser. Das zu behandelnde Wasser mit den enthaltenen Schadstoffen wird aus einem Brunnen 222 mittels einer Förderpumpe 223 gefördert und gelangt über eine Rohrleitung 224 bis zu einer Dosierstelle 204 für das Ozon. Die Dosierstelle 204 ist in diesem Ausführungsbeispiel als Injektor ausgestaltet, der sich oberhalb des Wasserniveaus des Reaktionsbeckens 206 befindet. Der Injektor 204 ist in Form einer Venturi-Düse gestaltet, so dass am Injektorausgang ein Gegendruck erzeugt wird, der eine Erhöhung der Wasserlöslichkeit des Ozongases bewirkt. Daran schließt sich eine turbulente Rohrstrecke 225 an, die in das Einlaufbecken 208 mündet. Die turbulente Rohrstrecke 225 ist dabei beispielsweise mindestens 10 m lang. Dabei ist die Rohrstrecke 225 so gestaltet, dass sich große Turbulenzen ergeben (Reynoldszahl > 5000). Durch diese Maßnahme wird eine optimale Lösung des Ozongases im Wasser erreicht. Messungen haben beispielsweise gezeigt, dass die Gasblasen, die im Bereich des Einlaufbeckens 208 aufsteigen, ozonfrei sind.

Im Bereich des Einlaufbeckens 208 befindet sich eine Einheit 220 zur Messung und gegebenenfalls Regelung des pH-Wertes. Der pH-Wert wird vorzugsweise in einem basischen Bereich eingestellt. Im Bereich des Einlaufbeckens 208 befindet sich eine Packung 212, die zur Adsorption der Ozonidsubstanz vorgesehen ist. Die Packung 212 besteht beispielsweise aus einer Kieselsteinschüttung oder aus metalloxidischen oder keramischen Materialien. Vorzugsweise weist die Packung oxidische Oberflächen auf, so dass auch hier eine Umsetzung des Ozons stattfinden kann. In dem Bereich des Einlaufbeckens 208 befindet sich die Dosierstelle 209 für die Ozonidsubstanz. Die Ozonidsubstanz wird beispielsweise mittels einer Zahnradpumpe 210 als ölige oder cremeartige Substanz in das Becken 208 eingebracht. Die Ozonidsubstanz ist nahezu wasserunlöslich und adsorbiert an der Packung 212. Im Einlaufbecken 208 befindet sich eine Zirkulationspumpe 207, die das Wasser durch die Packung 212 saugt und es in einen Rohrreaktor 205 einleitet. Das gepumpte Volumen der Zirkulationspumpe 207 ist dabei größerals das Pumpvolumen der Förderpumpe 223. Im stromabwärtigen Bereich des Rohrreaktors 205 kann ein weiterer Bereich mit einer Packung 232 für die Adsorption der Ozonidsubstanz vorgesehen sein. Durch die hohe Ausströmungsgeschwindigkeit aus dem Rohrreaktor 205 heraus findet im Reaktionsbecken 206 eine Zirkulationsbewegung statt, so dass die im Wasser enthaltenen Substanzen (Verunreinigungen) vollständig oxidiert und abgebaut werden können.

Vergleichbar mit der Ausgestaltung der Vorrichtung aus Fig. 1 ist ein Überlauf 214 für das behandelte Wasser vorgesehen, der in ein Überlaufbecken 215 mündet. Ein weiterer Überlauf 226 für das Wasser aus dem Reaktionsbecken 206 befindet sich zwischen dem Reaktionsbecken 206 und dem Einlaufbecken 208, so dass das überlaufende Wasser mehrfach die Packung 212 passiert.

Durch die ablaufenden oxidativen Prozesse kann es im Reaktionsbecken 206 zu einem Überschuss an Wasserstoffperoxid kommen. Das Wasserstoffperoxid fördert die Abbauprozesse und ist daher vorteilhaft. Das Wasserstoffperoxid kann durch den Überlauf 226 rezirkuliert werden, so dass es gleichmäßig zur Verfügung steht. Ein besonderer Vorteil hierbei ist, dass kein externes Wasserstoffperoxid zudosiert werden muss, so dass die potentiell gefährliche Lagerung und Dosierung von Wasserstoffperoxid entfällt. Falls die Wasserstoffperoxidkonzentration zu groß wird, kann die Ozondosierung gedrosselt werden.

Im Überlaufbecken 215 ist eine Einrichtung 216 für eine UV-Bestrahlung des Wassers vorgesehen, so dass überschüssiges Wasserstoffperoxid abgebaut werden kann, bevor es die Anlage verlässt. Nach dem Überlaufbecken 215 gelangt das Wasser über einen weiteren Überlauf anschließend in die Reinstwasserrinne 217. Hier und gegebenenfalls im Bereich des Überlaufs 214 kann eine Wasserstoffperoxidmessung 218 und eine Ozonmessung 219 stattfinden. Die Messwerte werden für eine Steuerung und/oder Regelung der Ozonzugabe und/oder der Ozonherstellung verwendet. Die Messwerte der Wasserstoffperoxidkonzentration am Überlauf 214 können auch für eine Steuerung der UV-Behandlung im Becken 215 eingesetzt werden. Überschüssiges Ozongas, das aus dem Wasser austritt, kann mit einem Ozonzerstörer 221 eliminiert werden.

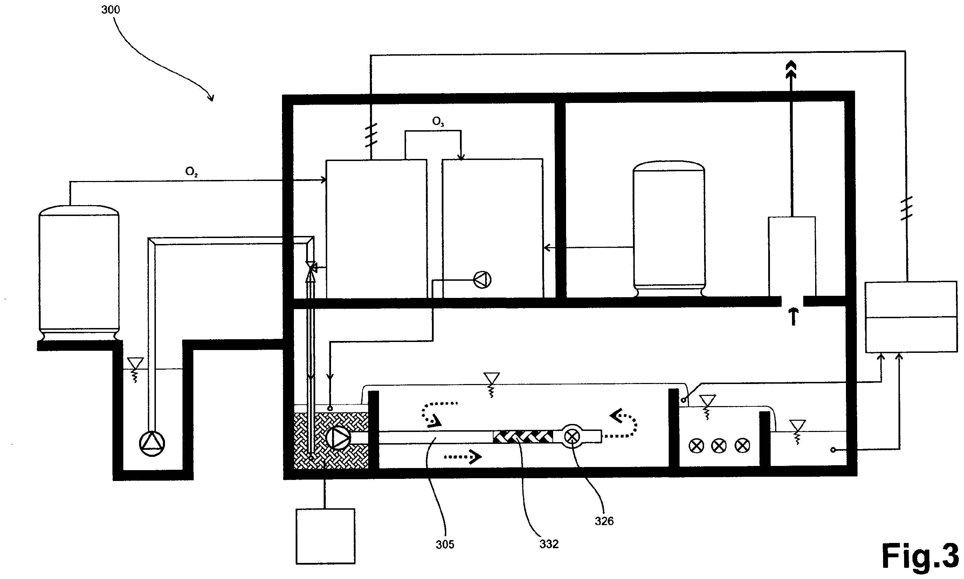

Fig. 3 zeigt ein weiteres Beispiel einer Vorrichtung 300 zur oxidativen Behandlung von belastetem Wasser. Die Vorrichtung 300 ist in weiten Teilen mit der Ausführungsform 200 vergleichbar, die anhand von Fig. 2 erläutert wurde. Im Unterschied hierzu weist die Ausführungsform 300 im stromabwärtigen Bereich des Rohrreaktors 305 eine weitere UV-Strahlungsquelle 326 auf. Der UV-Strahler 326 befindet sich stromabwärts der Packung 332, die zur Adsorption der Ozonidsubstanz vorgesehen ist. Durch die UV-Bestrahlung im Rohrreaktor wird die Bildung von Hydroxylradikalen aus Wasserstoffperoxid weiter verstärkt, so dass durch die UV-Bestrahlung an dieser Position der oxidative Abbau von Schadstoffen weiter verbessert wird.

Mit dem erfindungsgemäßen Verfahren, das insbesondere mittels der beschriebenen Vorrichtungen durchgeführt werden kann, kann vor allem organisch belastetes Wasser sehr effektiv aufbereitet werden. Verglichen mit einer Ozonbehandlung, wie sie an sich bekannt ist, erlaubt das erfindungsgemäße Verfahren, bei dem die zusätzliche oxidierende Komponente auf der Basis von ozonisierten Fettsäuren eingesetzt wird, einen wesentlich weitgehenderen Abbau und eine Mineralisierung der in dem Wasser enthaltenen organischen Substanzen, insbesondere auch einen Abbau von halogenierten organischen Kohlenwasserstoffen und anderen Spurenschadstoffen. Der erfindungsgemäße oxidative Abbau- oder Reinigungsprozess basiert auf einer radikalischen Kettenreaktion, wobei Hydroxylradikale die entscheidenden Moleküle sind. Durch die Kombination einer Ozonisierung des Wassers mit einer Behandlung mit der Komponente auf der Basis von ozonisierten Fettsäuren werden verschiedene Quellen für die Hydroxylradikal-Bildung bereitgestellt, die sich gegenseitig beeinflussen, so dass gerade diese Kombination die sehr effektive oxidative Behandlung bewirkt. Insbesondere sind dabei organische Ozonide die Initiatoren der radikalischen Kettenreaktion. Beim Kontakt mit Wasser zerfällt das im Wesentlichen wasserunlösliche Ozonid in die Bruchstücke Aldehyd und Carbonsäure, wobei sich Wasserstoffperoxid bildet (Gleichung G8). In Gegenwart von Ozon bildet sich letztendlich das Hydroxylradikal gemäß den folgenden Gleichungen, wobei die Gleichung G7 die Reaktionskonstante für die Reaktion des Anions mit Ozon zur Bildung des Hydroxylradikals beschreibt, die pH-Wert- und pKa-Wert-abhängig ist:

(G7) K(OH•) = K(HO₂⁻ + O₃) x 10^{(pH-pKa)}

(G7a) pKₐ (H₂O₂) = 11,6

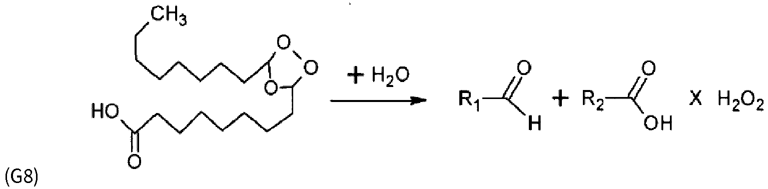

Damit bestehen für die Bildung des Hydroperoxid-Anions als Prekursor des Hydroxylradikals zunächst zwei Quellen, nämlich aus dem Zerfall von Ozon (Gleichung G1, siehe oben) und aus dem Abbau des Ozonids (Gleichung G8), insbesondere bei basischen pH-Werten. Auch die Carbonsäure, die aus dem organischen Ozonid entsteht, kann intermediär im Gesamtsystem ebenfalls Teil der radikalischen Kettenreaktion sein (ROO⁻) und eine weitere Quelle für die Hydroxylradikal-Bildung darstellen. Als weitere Quelle für die Hydroxylradikal-Bildung können erfindungsgemäß die oxidischen Oberflächen der Packungsmaterialien in der beschriebenen Vorrichtung wirken, an denen Ozon umgesetzt werden kann. Eine weitere Quelle für die Hydroxylradikal-Bildung kann durch die UV-Bestrahlung im Reaktionsbecken erzielt werden, wobei Wasserstoffperoxid unter UV-Einwirkung zu dem Hydroxylradikal umgesetzt wird.

Messungen zeigen, dass nach der Behandlung mit der Ozonidsubstanz gemäß der Erfindung im behandelten Wasser kein Ozon mehr feststellbar ist. Wasserstoffperoxid wird erst dann messbar, wenn für das Wasserstoffperoxid keine Reaktionsmöglichkeit mehr besteht, das heißt also erst dann, wenn die im Wasser enthaltenen organischen Substanzen vollständig abgebaut sind. Dies gilt vor allem bei einem pH-Wert von 8,4 oder mehr. Bei pH-Werten unterhalb von 8, beispielsweise bei pH 7,9, kann gegebenenfalls noch Ozon und Wasserstoffperoxid gemessen werden. Durch die Gegenwart von Ozonid verschwindet der Einfluss des pKa-Wertes (Gleichung G7a) auf die Stabilität des Ozons. Eine Überdosierung von Ozon in Gegenwart des aprotisch hergestellten Ozonids führt dann zu einem Anstieg des Wasserstoffperoxid-Gehalts. Dieses überschüssige Wasserstoffperoxid kann gegebenenfalls durch eine UV-Bestrahlung beseitigt werden.

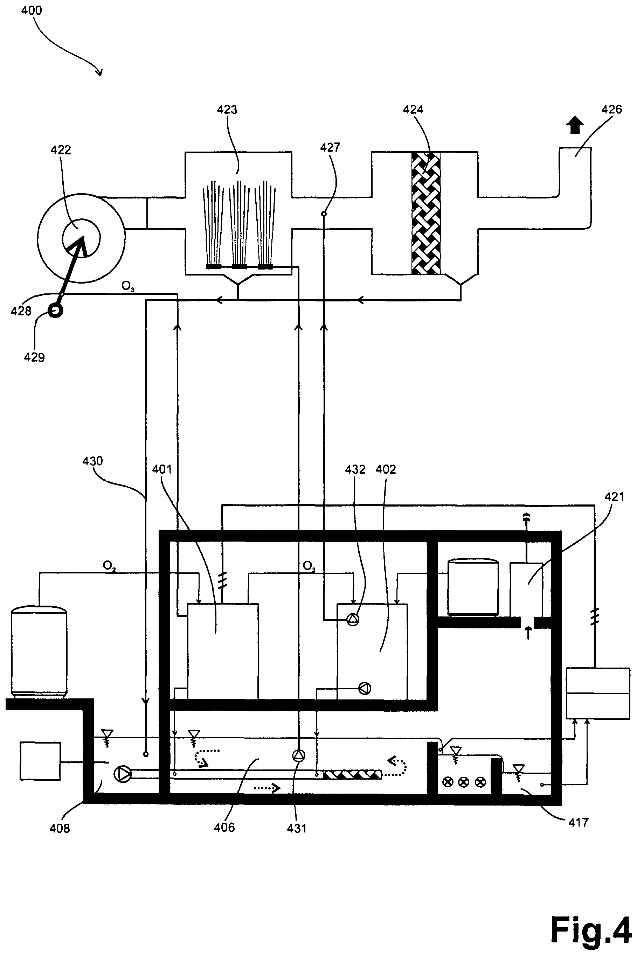

Fig. 4 illustriert eine Vorrichtung 400, bei der neben der oxidativen Wasserbehandlung eine weitergehende Behandlung der Abluft des Systems vorgesehen ist. Fig. 4 zeigt im unteren Teil die Vorrichtung für die eigentliche Wasserbehandlung, die im Wesentlichen der Ausführungsform entspricht, die in Fig. 1 gezeigt ist. Im oberen Teil der Abbildung ist der Abluftstrang der Anlage gezeigt. Bei der Abwasserbehandlung entstehen oftmals Geruchsemissionen, die insbesondere auf Schwefelverbindungen (z.B. Schwefelwasserstoff, Mercaptane) zurückzuführen sind.

Erfindungsgemäß werden diese geruchsintensiven Verbindungen durch eine Gaswäsche und/oder durch eine oxidative Behandlung im Abluftstrang beseitigt. Die Abluft aus der Wasserbehandlung, hier dargestellt durch den Pfeil 429, wird über einen Ventilator 422 in den nachfolgenden Abgasstrang eingespeist. Zunächst durchläuft das Abgas einen Gaswäscher 423. Der Gaswäscher 423 wird über eine Pumpe 431 mit Wasser aus dem Reaktionsbecken 406, das als Waschflüssigkeit im Gaswäscher 423 dient, gespeist. Dieses Wasser enthält infolge der oxidativen Prozesse bei der Wasserbehandlung Wasserstoffperoxid. Das Wasserstoffperoxid, das auf diese Weise in den Abluftstrang gelangt, befördert die oxidativen Prozesse bei der Abgasbehandlung. Nach der Gaswäsche gelangt das Abgas in einen Bereich mit einer Packung 424, die zur Absorption von Ozonid und/oder anderen peroxidischen Verbindungen, die durch eine Ozonisierung von ungesättigten Fettsäuren bereitgestellt werden, vorgesehen ist. Diese oxidierende Komponente wird über die Dosierstelle 427 in flüssiger oder in gasförmiger Form in den Abluftstrang eingebracht. Diese oxidierende Komponente wird beispielsweise in Form eines Ozonid-haltigen Gases mit einer Gaspumpe 432 aus der Einheit 402, die für die Bereitstellung der Ozonidsubstanz für die Wasserbehandlung vorgesehen ist, gefördert. Flüssigkeiten und Kondensate aus dem Gaswäscher 423 und aus dem Bereich mit der Packung 424 laufen über eine Drainageleitung 430 zurück in das oder die Becken der Abwasserbehandlung. In diesem Beispiel laufen die Flüssigkeiten zurück in das Wassereinlaufbecken 408. Durch die bei der oxidativen Behandlung gebildeten Produkte, insbesondere S02, ist das Kondensat im Allgemeinen sauer, abhängig von der Belastung der Abluft. Es kann daher auch zur Einstellung des pH-Wertes in der Anlage genutzt werden. Beispielsweise kann mit dem Kondensat der pH-Wert des auslaufenden Wassers eingestellt bzw. neutralisiert werden, indem das Kondensat in dem Bereich der Reinstwasserrinne 417 eingeleitet wird. Wenn erforderlich, kann das bei der oxidativen Behandlung gebildete S02 durch einen weiteren Gaswäscher ausgewaschen werden. Die gereinigte Abluft kann über den Ausgang 426 freigesetzt werden.

Ozon kann über die Ozondosierstelle 428 im Bereich des Ventilators 422 in den Abluftstrang dosiert werden. Dieses Ozon stammt aus dem Ozongenerator 401 der Wasserbehandlungsanlage. Alternativ oder zusätzlich kann beispielsweise die ozonhaltige Abluft aus dem Bereich des Ozonzerstörers 421 in den Abgasstrang eingespeist werden. Bei einer solchen Ausgestaltung kann gegebenenfalls auf den Ozonzerstörer 421 verzichtet werden. Die erfindungsgemäße Abluftbehandlung beruht im Prinzip darauf, dass das Abgas in Form von feuchtem Ozongas eine Oberfläche mit Ozonid und/oder anderen peroxidischen Verbindungen passiert. Die Befeuchtung des mit Ozon angereicherten Abgases wird hierbei vorzugsweise durch eine Gaswäsche realisiert, wobei der Gaswäscher mit peroxidhaltigem Wasser aus der Wasserbehandlung betrieben wird.

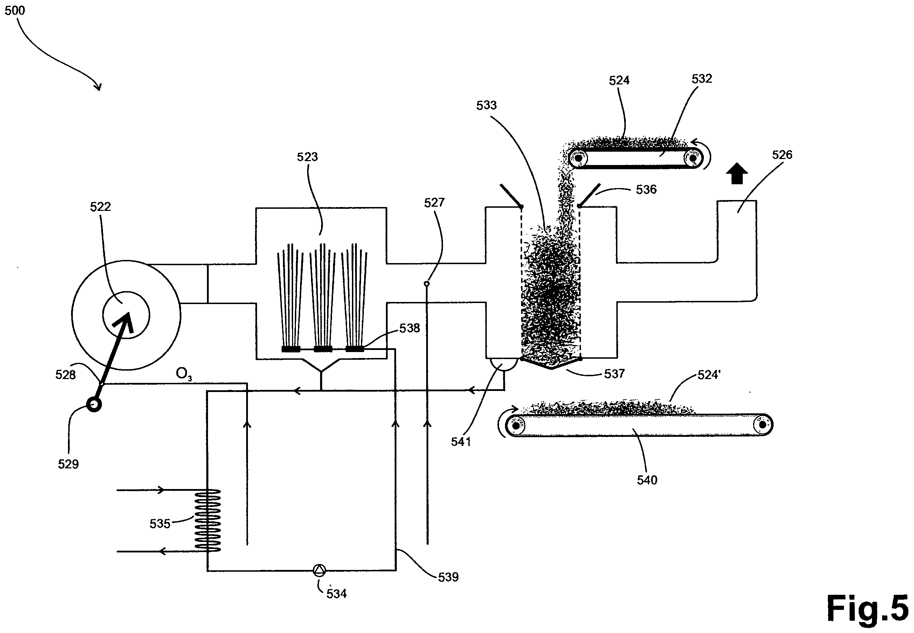

Fig. 5 zeigt eine beispielhafte Ausgestaltung einer Vorrichtung 500 zur erfindungsgemäßen Behandlung von Feststoffen, die in diesem Beispiel zerkleinert sind. Insbesondere kann es sich hierbei um geschredderte Abfälle, z.B. um Krankenhausmüll, handeln. Über ein Transportband 532 wird zerkleinerter Müll 524 in einen Behandlungsbehälter 533 eingebracht. Der Einlass und der Auslass des Behandlungsbehälters 533 sind durch Klappen 536 bzw. 537 verschließbar. Der Behandlungsbehälter 533 wird von einem angefeuchteten Luftstrom, der mit Ozon und mit Ozonid angereichert ist, durchströmt. Hierfür ist im Bereich der Luftzufuhr529 der Vorrichtung 500 eine Ozondosierstelle 528 vorgesehen, wobei die einströmende Luft mit Ozon angereichert wird. Die in die Vorrichtung zugeführte Luft 529 wird mittels eines Ventilators 522 angetrieben. Der Luftstrom durchläuft eine Befeuchtungseinheit 523. In der Befeuchtungseinheit 523 sind Wasserdüsen 538 vorgesehen, die eine Vemebelung von Wasser bewirken. Die Wasserdüsen 538 werden über einen Wasserkreislauf 539 gespeist, der mit einer Pumpe 534 angetrieben wird. Der mit Ozon angereicherte und befeuchtete Luftstrom passiert nachfolgend eine Dosierstelle 527 für Ozonid. Der Luftstrom wird dabei zusätzlich mit Ozonid oder allgemein mit einer Komponente, die durch die Ozonisierung von wenigstens einem Olefin bereitgestellt wird, angereichert. Diese oxidierende Komponente kann in flüssiger oder in gasförmiger Form eingebracht werden. Anschließend gelangt der Luftstrom in den Behandlungsbehälter 533 mit den zu behandelnden Abfällen. Die mitgeführte Ozonid-Komponente wird an den Feststoffen adsorbiert und die beschriebenen oxidativen Prozesse können in dem herrschenden feuchten Milieu ablaufen. Die dermaßen behandelten Abfälle 524 werden über die Klappen 537 am Auslass des Behälters 533 ausgeworfen und können über ein Transportband 540 abtransportiert werden. Überschüssige Flüssigkeit in dem Behandlungsbehälter 533 sammelt sich an einem Wasserauslass 541 des Behandlungsbehälters 533 und wird wieder dem Wasserkreislauf 539 zugeführt. Nach dem Durchlaufen des Behälters 533 verlässt die Luft die Vorrichtung über eine Abluftöffnung 526.

Durch die Verdunstung des Wassers im Bereich der Befeuchtungseinheit 523 kühlt das Wasser im Wasserkreislauf 539 ab. Diese Verdunstungskälte kann mit einem Wärmetauscher 535 genutzt werden, um andere Medien abzukühlen (indirekter Verdunstungskühler). Die hierbei eintretende Erwärmung des Wassers in dem Wasserkreislauf 539 ist darüber hinaus für die effektive Befeuchtung des Luftstroms im Bereich der Befeuchtungseinheit 523 vorteilhaft.

Alternativ zu dem in Fig. 5 gezeigten Behandlungsbehälter 533 und den Transportbändern 532 und 540 ist in vergleichbarer Weise beispielsweise auch ein kontinuierlicher Betrieb mit einem Zyklon möglich.

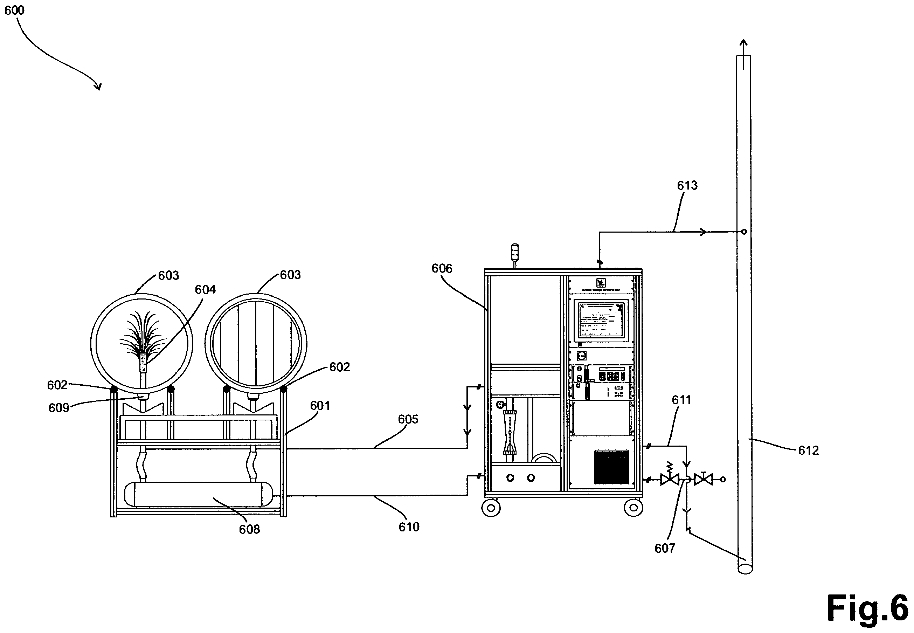

Fig. 6 illustriert eine Vorrichtung 600 zur oxidativen (sterilisierenden) Behandlung der inneren Oberflächen von Weinfässern 603 oder von Tanks auf der Basis des erfindungsgemäßen Verfahrens. Auf einem Lagergestell 601 mit Positionsrollen 602 sind die zu reinigenden Fässer 603 oderTanks gelagert. In das Innere der Fässer 603 ragt durch eine entsprechende Öffnung in den Fässern 603 oder Tanks ein Sprühkopf 604. Im Verlauf der Behandlung wird über den Sprühkopf 604 eine Ozonidsubstanz in das Innere des Fasses 603 oder des Tanks eingesprüht. Anschließend, gleichzeitig oder zuvor wird Ozonwasser, also mit Ozon versetztes Wasser, oder Ozongas über den Sprühkopf 604 in das Innere des Fasses 603 oder des Tanks eingebracht. Zu diesem Zweck steht der Sprühkopf 604 über eine Schlauchverbindung 605 als Zuführleitung mit einer Versorgungseinheit 606 in Verbindung. Über die Versorgungseinheit 606 erfolgen die Bereitstellung und die Zufuhr von ozonisiertem Wasser (oder gegebenenfalls Ozongas) und der Ozonidsubstanz über die Schlauchverbindung 605. Die Versorgung der Versorgungseinheit 606 mit Frischwasser erfolgt über eine Frischwasserzufuhr 607. Im unteren Bereich des Lagergestells 601 ist ein Sammelbehälter 608 für Flüssigkeiten aus den Fässern 603 oder Tanks vorgesehen. Sobald die oxidativen Prozesse innerhalb der Fässer 603 oder Tanks abgeschlossen sind, kann über eine Absaugung 609 die in den Fässern 603 oder Tanks enthaltende Flüssigkeit abgesaugt und im Sammelbehälter 608 aufgefangen werden. Die Flüssigkeit aus dem Sammelbehälter 608 wird über eine Schlauchverbindung 610 als Abführleitung in die Versorgungseinheit 606 geleitet und von dort über einen Ablauf 611 in einen Entsorgungskanal 612 entsorgt. Die entstehenden Abgase können über eine Entlüftungsleitung 613 ebenfalls entsorgt werden.

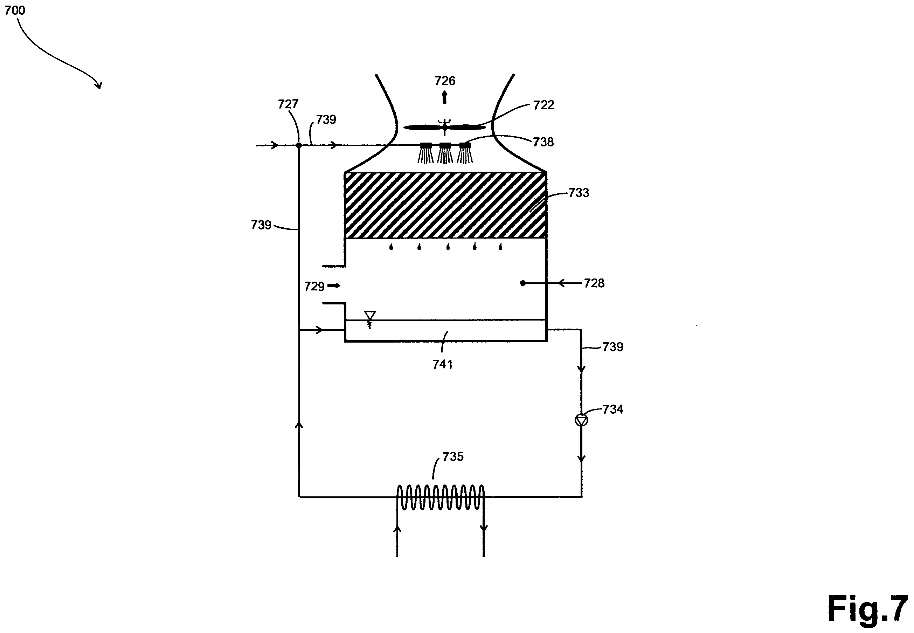

Fig. 7 zeigt einen Verdunstungskühler 700, der zur Kühlung eines Luftstromes vorgesehen ist. Dieser Verdunstungskühler kann beispielsweise Bestandteil einer Klimaanlage sein. Der Luftstrom ist durch Pfeile 729 und 726 angedeutet, wobei die Luft über eine Luftzufuhr 729 in den Verdunstungskühler 700 eintritt und über den Luftaustritt 726 den Verdunstungskühler 700 verlässt. Die Luft durchläuft dabei eine Packung 733, beispielsweise eine Kunststoffpackung. Die Packung 733 wird mittels einer Mehrzahl von Düsen 738 mit Wasser besprüht, so dass die Packung 733 mit Feuchtigkeit benetzt wird. Durch die Verdunstung des Wassers insbesondere im Bereich der Packung 733 wird eine Kühlwirkung für die die Packung 733 durchströmende Luft erzielt. Die Verdunstung des Wassers kann durch einen Ventilator 722 unterstützt werden. Im unteren Bereich des Verdunstungskühlers ist ein Wasserbehälter 741 zum Auffangen von überschüssiger Flüssigkeit vorgesehen. Der Wasserbehälter 741 ist Teil eines Wasserkreislaufes 739, der mittels einer Pumpe 734 angetrieben wird und der die Düsen 738 speist. Durch die Verdunstung von Wasser im Bereich der Packung 733, also innerhalb des Wasserkreislaufes 739, kühlt sich das Kreislaufwasser ab und kann zu Kühlzwecken für einen Wärmetauscher 735 genutzt werden. Für die erfindungsgemäße oxidative Behandlung des Verdunstungskühlers 700 ist zum einen eine Ozondosierstelle 728 zur Einspeisung von Ozongas oder von ozonisiertem Wasser in den Luftstrom vorgesehen. Zum anderen ist eine Ozoniddosierstelle 727 für die Substanz, die durch die Ozonisierung von wenigstens einem Olefin, also für die Ozonidsubstanz, vorgesehen, wobei die Ozonidsubstanz in der hier illustrierten Ausgestaltung in den Wasserkreislauf eingespeist wird und über die Düsen 738 auf der Packung 733 verteilt wird. Die kombinierte Behandlung mit Ozon und Ozonid führt zu den beschriebenen oxidativen Prozessen und Kettenreaktionen und damit zu einer Hygienisierung des Verdunstungskühlers 700, so dass eine bakterielle Kontamination und Veralgung insbesondere im Bereich der Packung 733 zuverlässig vermieden wird und ein sauberer und gleichzeitig gekühlter Luftstrom erreicht wird.

## Patentansprüche

1. Verfahren zur oxidativen Behandlung einer Flüssigphase und/oder einer Gasphase und/oder einer Festphase, **dadurch gekennzeichnet, dass** für die Behandlung Ozon und wenigstens ein in einem separaten Prozess aprotisch hergestelltes, als cremeartige oder ölartige Substanz vorliegendes organisches Ozonid zugesetzt werden, wobei das Verfahren in Gegenwart von Wasser durchgeführt wird, so dass das Ozonid, das Ozon und das Wasser so reagieren, dass Hydroxylradikale gebildet werden, die in einer Kettenreaktion unselektiv organische Bestandteile der zu behandelnden Phase bis zu deren höchster Oxidationsstufe oxidieren.

2. Verfahren nach Anspruch 1, **dadurch gekennzeichnet, dass** für eine Adsorption des wenigstens einen organischen Ozonids eine Packung bereitgestellt wird.

3. Verfahren nach Anspruch 1 oder Anspruch 2, **dadurch gekennzeichnet, dass** die Flüssigphase belastetes Wasser ist, wobei das Wasser ozonisiert und mit dem wenigstens einen organischen Ozonid behandelt wird.

4. Verfahren nach Anspruch 3, **dadurch gekennzeichnet, dass** zur Behandlung der Abluft aus dem Verfahren die Abluft mit wenigstens einem organischen Ozonid behandelt wird und wobei vorzugsweise die Abluft ozonisiert wird.

5. Verfahren nach Anspruch 1 oder Anspruch 2, **dadurch gekennzeichnet, dass** die Gasphase ein Abgas und/oder Abluft ist.

6. Verfahren nach Anspruch 1 oder Anspruch 2, **dadurch gekennzeichnet, dass** die Festphase von inneren Oberflächen von Fässern, insbesondere Holzfässern, oder Tanks gebildet wird.

7. Verfahren nach Anspruch 1 oder Anspruch 2, **dadurch gekennzeichnet, dass** die Festphase von Abfällen, insbesondere Krankenhausabfällen, gebildet wird, wobei vorzugsweise die Abfälle vor der oxidativen Behandlung zerkleinert werden.

## Claims

1. Method for the oxidative treatment of a liquid phase and/or a gas phase and/or a solid phase, **characterized in that** ozone and at least one organic ozonide, prepared aprotically in a separate operation and taking the form of a cream-like or oil-like substance, are added for the treatment, the method being carried out in the presence of water and so the ozonide, the ozone and the water reacting such that hydroxyl radicals are formed which in a chain reaction unselectively oxidize organic constituents of the phase that is to be treated to their highest oxidation state.

2. Method according to Claim 1, **characterized in that** a packing is provided for adsorption of the at least one organic ozonide.

3. Method according to Claim 1 or Claim 2, **characterized in that** the liquid phase is polluted water, the water being ozonized and treated with the at least one organic ozonide.

4. Method according to Claim 3, **characterized in that** for the treatment of the exhaust air from the method, the exhaust air is treated with at least one organic ozonide, the exhaust air preferably being ozonized.

5. Method according to Claim 1 or Claim 2, **characterized in that** the gas phase is an exhaust gas and/or exhaust air.

6. Method according to Claim 1 or Claim 2, **characterized in that** the solid phase is formed by internal surfaces of barrels, especially wooden barrels, or tanks.

7. Method according to Claim 1 or Claim 2, **characterized in that** the solid phase is formed by wastes, especially hospital wastes, the wastes preferably being comminuted before the oxidative treatment.

## Revendications

1. Procédé de traitement oxydatif d'une phase liquide et/ou d'une phase gazeuse et/ou d'une phase solide, **caractérisé en ce que** de l'ozone et au moins un ozonure organique fabriqué par voie aprotique dans un processus séparé, qui se présente sous la forme d'une substance crémeuse ou huileuse, sont ajoutés pour le traitement, le procédé étant réalisé en présence d'eau, de telle sorte que l'ozonure, l'ozone et l'eau réagissent pour former des radicaux hydroxyle qui oxydent de manière non sélective des constituants organiques de la phase à traiter jusqu'à leur niveau d'oxydation le plus élevé par une réaction en chaîne.

2. Procédé selon la revendication 1, **caractérisé en ce qu'**un garnissage est préparé pour une adsorption dudit au moins un ozonure organique.

3. Procédé selon la revendication 1 ou la revendication 2, **caractérisé en ce que** la phase liquide est de l'eau chargée, l'eau étant ozonisée et traitée avec ledit au moins un ozonure organique.

4. Procédé selon la revendication 3, **caractérisé en ce que**, pour le traitement de l'air d'échappement du procédé, l'air d'échappement est traité avec au moins un ozonure organique, l'air d'échappement étant de préférence ozonisé.

5. Procédé selon la revendication 1 ou la revendication 2, **caractérisé en ce que** la phase gazeuse est un gaz d'échappement et/ou un air d'échappement.

6. Procédé selon la revendication 1 ou la revendication 2, **caractérisé en ce que** la phase solide est formée par des surfaces intérieures de fûts, notamment de fûts en bois, ou de réservoirs.

7. Procédé selon la revendication 1 ou la revendication 2, **caractérisé en ce que** la phase solide est formée par des déchets, notamment des déchets hospitaliers, les déchets étant de préférence broyés avant le traitement oxydatif.
